# EUROPEAN PATENT SPECIFICATION

(11) **EP 4 458 259 B1**
(45) Date of publication and mention of the grant of the patent: **06.05.2026**
(21) Application number: 23844896.3
(22) Date of filing: 10.04.2023
(51) Int. Cl.: A61B 5/021, A61B 5/022, A61B 5/024, A61B 5/00

(54) **BLOOD PRESSURE MEASUREMENT METHOD FOR WEARABLE DEVICE AND WEARABLE DEVICE**
BLUTDRUCKMESSVERFAHREN FÜR WEARABLE-VORRICHTUNG UND WEARABLE-VORRICHTUNG
PROCÉDÉ DE MESURE DE PRESSION ARTÉRIELLE POUR DISPOSITIF PORTABLE ET DISPOSITIF PORTABLE

(30) Priority: 25.07.2022 CN 202210875921
(43) Date of publication of application: 06.11.2024
(73) Proprietor: Honor Device Co., Ltd., Shenzhen, Guangdong 518040 (CN)
(72) Inventor: SU, Dan, Shenzhen, Guangdong 518040 (CN); LIU, Yi, Shenzhen, Guangdong 518040 (CN); CHONG, Hao, Shenzhen, Guangdong 518040 (CN)
(74) Representative: Isarpatent
(86) International application number: PCT/CN2023/087370
(87) International publication number: WO 2024/021679

(56) References cited:
- WO-A1-2019/169636
- CN-A- 110 141 197
- CN-A- 113 440 118
- CN-A- 113 712 517
- CN-A- 114 073 520
- US-A1- 2017 020 399
- US-A1- 2018 184 920
- US-A1- 2019 008 399
- US-A1- 2020 093 377
- US-A1- 2020 113 442
- US-A1- 2022 047 170

## Description

This application claims priority to Chinese Patent Application No. 202210875921.7, filed with the China National Intellectual Property Administration on July 25, 2022 and entitled "BLOOD PRESSURE MEASUREMENT METHOD OF WEARABLE DEVICE, AND WEARABLE DEVICE".

### TECHNICAL FIELD

This application relates to the field of terminal technologies, and in particular, to a blood pressure measurement method of a wearable device, and a wearable device.

### BACKGROUND

With the development of wearable devices, the wearable device may support increasingly more functions. To satisfy needs of users for health management thereof, a relatively large quantity of wearable devices may support a human body data monitoring function of the user. For example, a smartwatch may measure a human body feature such as a heart rate, a respiration rate, blood pressure, or blood oxygen.

Currently, for measurement of the blood pressure, a method for obtaining a blood pressure value based on a photoplethysmography (photoplethysmography, PPG) signal of a fingertip and a fingertip pressure exists on the market. The user applies a pressure to the wearable device through the fingertip, and the wearable device obtains the pressure of the fingertip through a pressure transducer (pressure transducer, PT), and emits an optical signal through a light emitting diode (light emitting diode, LED) in a PPG module. The optical signal is absorbed and scattered by skin tissue of the fingertip to form a reflected signal. The reflected signal may be received by a photo diode (photo diode, PD) in the PPG module and converted into a PPG signal. The wearable device may obtain the blood pressure value based on a waveform of the PPG signal of the fingertip.

However, the method has a problem that the blood pressure value obtained through measurement is inaccurate.

US 2019/008399 A1 describes a system and method for cuff-less blood pressure measurement in a mobile device. A key aspect of this disclosure is the discovery of a new location for blood pressure measurement at the fingertip of a subject and that reflectance-mode photoplethysmography can be used to help make this measurement. Through experiments in human subjects, it was discovered that it is indeed possible to measure systemic blood pressure by having a subject press the fingertip against a reflectance-mode photo-plethysmography-force sensor unit under visual guidance and then compute blood pressure from the resulting variable-amplitude blood volume oscillations and applied pressure via an oscillometric algorithm.

US 2020/093377 A1 describes an apparatus for estimating bio-information. The apparatus for estimating bio-information includes: a sensor part comprising a pulse wave sensor array configured to detect a pulse wave signal when an object contacts a contact surface of the sensor part, and a load sensor configured to detect a first contact load applied by the object to the contact surface; and a processor configured to obtain contact load distribution of the contact surface based on the pulse wave signal, and to estimate bio-information based on the contact load distribution.

US 2020/113442 A1 describes blood pressure detection apparatuses and methods for detecting a blood pressure of a user comprising optical sensors/detectors and a force sensor and, in some embodiments, comprising only a force sensor, measuring force applied by a finger. Blood pressure is measured by applying an increasing (or decreasing) force or pressure with a finger of the user on at least the force sensor, which, in some embodiments, may be a plurality of increasing pressure steps, each step being held within a predetermined acceptable pressure/force tolerance range for a predetermined hold time, and measuring the force applied by the finger and, in some embodiments, optically measuring the blood in a vessel in the finger relating to the applied pressure/force. Feedback (visual, haptic, sound) of the applied pressure is provided to the user.

CN 110 141 197 A describes electric equipment with a display screen. The electric equipment with the display screen is used for obtaining physiological characteristics and/or pathological characteristicsby acquiring the biometric information of a user. The electric equipment with the display screen comprises a PPG light receiving unit and a PPG obtaining unit, wherein the PPG light receiving unit isused for receiving a light signal and converting the light signal into a pulse wave signal; the PPG obtaining unit is used for receiving and processing the pulse wave signal from the PPG light receiving unit to obtain physiological characteristics and/or pathological characteristics; the display screen is arranged in a manner that the reflected part of the light emitted by the display screen to the first side can penetrate through the display screen to reach the second side opposite to the first side, and the reflected part of the light is received by the PPG light receiving unit arranged on the second side, so that the electric equipment with the display screen can acquire the biometric information of the user based on a pulse wave photo plethysmo graphy.

US 2022/047170 A1 describes a display device that includes: a display panel having a plurality of light emitting elements to display an image, the display panel including a first area; a blood pressure sensor disposed adjacent to the display panel to sense pressure applied to at least a portion of the first area and to sense light incident thereon; and a pressure transmission member overlapping the first area of the display panel and the blood pressure sensor.

### SUMMARY

An object of the present invention is to provide a blood pressure measurement method of a wearable device, and a wearable device, so as to improve accuracy of a blood pressure value. This object is solved by the subject-matter of the appended independent claims. Further embodiments are defined in the dependent claims.

According to a first aspect, a blood pressure measurement method of a wearable device is provided. The wearable device includes a pressure transducer PT, a display screen, and a photo diode PD. The method includes: emitting an optical signal through a first region of a display screen; converting, into a first PPG signal, the optical signal received by the PD; emitting the optical signal through a second region of the display screen if the first PPG signal does not satisfy a signal quality requirement, where the second region and the first region are different in at least one of the following features: a position, luminance, an area size, or a shape on the display screen; converting, into a second PPG signal, the optical signal received by the PD; obtaining a pressure value collected by the PT when the second PPG signal satisfies the signal quality requirement; and determining a blood pressure value of a wearer based on the pressure value and the second PPG signal.

The display screen may also be referred to as a display screen or a screen. This is not limited in this application. The first region of the display screen is a light emitting region or a light emitting region of the screen in embodiments. It may be understood that the first region is smaller than or equal to a region of the display screen.

When the wearer touches or presses the first region with a finger, the optical signal emitted by the first region may be absorbed and scattered by skin tissue of the finger to form a reflected signal, and the PD may convert the reflected signal into the first PPG signal. The first PPG signal is an initial PPG signal in embodiments.

If the first PPG signal does not satisfy the signal quality requirement, the wearable device may obtain the second region by adjusting at least one of the position of the first region on the display screen, the luminance of the display screen in the first region, the area size of the first region, or the shape of the first region, and emit the optical signal by using the second region, to obtain a PPG signal that satisfies the signal quality requirement.

If the second PPG signal satisfies the signal quality requirement, the wearable device may collect one or more second PPG signals. This is not limited in this application.

A collection moment of the pressure value is the same as a collection moment of the second PPG signal. When one second PPG signal is collected, one pressure value is also obtained. When a plurality of second PPG signals are collected, a plurality of pressure values are also obtained.

The wearable device may determine the blood pressure value of the wearer based on the collected PPG signal and the pressure value.

According to the blood pressure measurement method of a wearable device provided in this application, the second region is obtained by adjusting at least one of the position of the first region on the display screen, the luminance of the display screen in the first region, the size of the first region, or the shape of the first region, to obtain the second PPG signal that satisfies the signal quality requirement. The PPG signals configured to measure the blood pressure value are all signals that satisfy the signal quality requirement, which is advantageous in improving measurement accuracy of the blood pressure value. In addition, in this application, the first region of the display screen is used as a measurement light source, and the measurement light source does not need to be separately deployed, which is advantageous in saving space of the wearable device and advantageous for application and promotion.

With reference to the first aspect, in some implementations of the first aspect, that the first PPG signal does not satisfy a signal quality requirement includes: a perfusion rate of the first PPG signal is less than or equal to a preset perfusion rate; and a distance between a projection region of the PD on the display screen and the second region is greater than a distance between the projection region of the PD on the display screen and the first region.

A first distance is defined between the projection region of the PD on the display screen and the first region. In other words, the first region and the PD are staggered by a specific distance in a horizontal direction. This is not limited in this application.

If the first PPG signal does not satisfy the signal quality requirement due to the low perfusion rate of the first PPG signal, the wearable device may increase a horizontal distance between the first region and the PD. A position of the PD may be fixed, and the wearable device may change the position of the first region on the display screen to obtain the second region.

According to the blood pressure measurement method of a wearable device provided in this application, the perfusion rate of the PPG signal is increased by increasing the distance between the projection region of the PD on the display screen and the first region, which is advantageous in obtaining the PPG signal that satisfies the signal quality requirement and advantageous in improving the measurement accuracy of the blood pressure value.

With reference to the first aspect, in some implementations of the first aspect, that the first PPG signal does not satisfy a signal quality requirement includes: an intensity of the first PPG signal is less than or equal to a preset intensity; and the luminance of the second region is greater than the luminance of the first region.

If the first PPG signal does not satisfy the signal quality requirement due to a too weak first PPG signal, that is, the intensity of the first PPG signal is less than or equal to the preset intensity, the wearable device may emit light through the second region of the display screen. The position of the second region is the same as that of the first region, but the luminance thereof is greater than that of the first region. In a possible implementation, the wearable device may increase the luminance of the display screen based on a preset increment.

According to the blood pressure measurement method of a wearable device provided in this application, the intensity of the PPG signal is increased by increasing the luminance of the display screen in the first region, which is advantageous in obtaining the PPG signal that satisfies the signal quality requirement and advantageous in improving the measurement accuracy of the blood pressure value.

With reference to the first aspect, in some implementations of the first aspect, the method further includes: emitting the optical signal through a third region of the display screen if the second PPG signal does not satisfy the signal quality requirement, where a distance between a projection region of the PD on the display screen and the third region is less than a distance between the projection region of the PD on the display screen and the first region; converting, into a third PPG signal, the optical signal received by the PD; obtaining a pressure value collected by the PT if the third PPG signal satisfies the signal quality requirement; and determining the blood pressure value of the wearer based on the pressure value and the third PPG signal.

If the second PPG signal cannot satisfy the signal quality requirement through luminance adjustment, that is, the intensity of the second PPG signal is still less than or equal to the preset intensity when the luminance of the second region is the maximum value, the wearable device may reduce the horizontal distance between the first region and the PD. The position of the PD may be fixed, and the wearable device may change the position of the first region on the display screen to obtain the third region. The distance between the projection region of the PD on the display screen and the third region is less than the distance between the projection region of the PD on the display screen and the first region.

According to the blood pressure measurement method of a wearable device provided in this application, the intensity of the PPG signal is increased by reducing an optical path when the luminance of the first region reaches the maximum value, which is advantageous in obtaining the PPG signal that satisfies the signal quality requirement and advantageous in improving the measurement accuracy of the blood pressure value.

With reference to the first aspect, in some implementations of the first aspect, the shape of the second region is different from the shape of the first region.

The shape of the first region belongs to a shape set. The shape set includes a circle, an ellipse, a bar, a square, a triangle, or an arc. The wearable device may change the shape of the first region to any shape in the shape set other than the shape of the first region, to obtain the second region.

With reference to the first aspect, in some implementations of the first aspect, the area size of the second region is different from the area size of the first region.

The wearable device may change the area size of the first region based on a preset zooming criterion to obtain the area size of the second region.

The preset zooming criterion may include a zoom-out criterion or a zoom-in criterion.

If the PPG signal does not satisfy the signal quality requirement, the wearable device may continuously change the size of the first region based on the preset zooming criterion until the PPG signal that satisfies the signal quality requirement is obtained.

According to the blood pressure measurement method of a wearable device provided in this application, the PPG signal that satisfies the signal quality requirement is obtained by changing the area size of the first region and reducing or enlarging the light emitting region, which is advantageous in improving the measurement accuracy of the blood pressure value.

With reference to the first aspect, in some implementations of the first aspect, the pressure value includes a plurality of pressure values, the second PPG signal includes a plurality of second PPG signals, the plurality of pressure values are in a one-to-one correspondence with the plurality of second PPG signals, and a collection moment of each of the plurality of pressure values is the same as that of a corresponding one of the second PPG signals. The determining a blood pressure value of a wearer based on the pressure value and the second PPG signal includes: extracting a waveform feature of each of the plurality of second PPG signals; and determining, if a target second PPG signal whose waveform feature is less than or equal to a preset waveform feature exists in the plurality of second PPG signals, a pressure value corresponding to the target second PPG signal as a systolic blood pressure (systolic blood pressure, SBP) in the blood pressure value of the wearer.

When different pressure values exist between the wearable device and a contact part, the wearable device may collect a plurality of pressure values through the PT. The plurality of pressure values may be generated by the wearer by pressing, but this application is not limited thereto. The wearer may also be referred to as a user. This is not limited in this application.

The wearable device may collect the second PPG signal under each pressure value to obtain the plurality of second PPG signals when collecting each of the plurality of pressure values. In other words, the plurality of pressure values are in a one-to-one correspondence with the plurality of second PPG signals, and the collection moment of each of the plurality of pressure values is the same as that of the corresponding second PPG signal.

The wearable device may respectively extract the waveform features of the plurality of second PPG signals through a feature extraction model, and may compare the waveform features of the plurality of second PPG signals with the preset waveform feature. If a target second PPG signal whose waveform feature is less than or equal to a preset waveform feature exists, the pressure value corresponding to the target second PPG signal is determined as the SBP. The preset waveform feature is configured for representing that no apparent waveform feature exists. In other words, a researcher may determine the preset waveform feature based on the waveform features of a large quantity of PPG signals that do not have apparent waveform features. It may be understood that the target second PPG signal whose waveform feature is less than or equal to the preset waveform feature does not have an apparent waveform feature.

If the target second PPG signal does not have an apparent waveform feature, it may indicate that the pressure value is relatively large in this case. A pressure between the wearable device and the contact part may cause blood vessels in the contact part to squeeze and form occlusion, and the systolic blood pressure may be accurately measured in this case.

According to the blood pressure measurement method of a wearable device provided in this application, the pressure value corresponding to the target second PPG signal whose waveform feature is less than or equal to the preset waveform feature is determined as the SBP, and the systolic blood pressure may be directly obtained through the PT. This is simple, efficient, and highly accurate.

With reference to the first aspect, in some implementations of the first aspect, the pressure value includes a plurality of pressure values, the second PPG signal includes a plurality of second PPG signals, the plurality of pressure values are in a one-to-one correspondence with the plurality of second PPG signals, and the collection moment of each of the plurality of pressure values is the same as that of the corresponding second PPG signal. The determining a blood pressure value of a wearer based on the pressure value and the second PPG signal includes: extracting a waveform feature of each of the plurality of second PPG signals; inputting the plurality of pressure values and the waveform features of the plurality of second PPG signals into a neural network model, where the neural network model is trained based on a historical pressure value and a waveform feature of a historical PPG signal, and is configured to measure blood pressure; and determining output of the neural network model as a diastolic blood pressure DBP in the blood pressure value of the wearer.

The researcher may train the neural network model based on the historical pressure value and the waveform feature of the historical PPG signal to determine the DBP in the blood pressure value.

The wearable device may use the plurality of pressure values and the waveform features of the plurality of PPG signals as the input to the neural network model, and determine the output of the neural network model as the DBP.

Optionally, the wearable device may use a pressure value of the plurality of pressure values that is less than or equal to the pressure value corresponding to the target second PPG signal (that is, the PPG signal that does not have an apparent waveform feature) and the PPG signal corresponding to the pressure value as the input to the neural network model, and determine the output of the neural network model as the DBP.

According to the blood pressure measurement method of a wearable device provided in this application, the diastolic blood pressure is calculated through the waveform feature presented by the pressure value and the second PPG signal. Compared with the blood pressure value calculated through regression or another calculation method, accuracy of the blood pressure value is improved.

With reference to the first aspect, in some implementations of the first aspect, before the obtaining a pressure value collected by the PT, the method further includes: outputting an operation guide, where the operation guide is configured to guide the wearer to apply a pressure perpendicular to a contact surface between the wearable device and human skin to the wearable device in a preset pressing region, and the operation guide includes a pressure value required for the wearer to press.

The pressure value between the wearable device and the contact part may be generated by the wearer by pressing based on the operation guide. The wearable device may guide the user to continuously apply a pressure to the wearable device through texts, sound, an image, or a color. In other words, the operation guide may be the texts, the sound, the image, or the color. This is not limited in this application. In a possible implementation, the operation guide may be "please increase the pressing pressure". The wearable device may guide the user through the sound and/or the texts. In another possible implementation, the wearable device displays a pressure curve through a human-machine interaction interface, and guides the user to continuously apply the pressure to the wearable device based on the pressure curve.

The wearable device may guide the user to continuously apply the pressure to the wearable device over a period of time based on the operation guide. The continuously applied pressure is perpendicular to the contact surface between the wearable device and the human skin (the contact part or the tested part), so that a continuous pressure change is generated between the wearable device and the human skin. A duration of the continuous pressure application is not limited in embodiments of this application. In a possible implementation, the duration for the continuous pressure application may be 30 seconds.

It is to be noted that the process of continuous pressure application is a process of increasing the pressure. In this embodiment of this application, the blood vessels in the skin in contact with the wearable device are squeezed to form the occlusion through the continuous pressure application by the user, so as to measure the blood pressure.

According to the blood pressure measurement method of a wearable device provided in this application, the wearer is guided to apply the pressure through the operation guide, so as to regulate a pressure application behavior of the wearer. The applied pressure is controllable and is an expected pressure value, which is advantageous for measurement of a subsequent blood pressure value.

With reference to the first aspect, in some implementations of the first aspect, the method further includes: ending, during the pressure application by the wearer, blood pressure measurement if the following condition is satisfied: an acceleration of the wearable device is greater than an acceleration threshold; and/or an angular velocity of the wearable device is greater than an angular velocity threshold.

During the pressure application by the wearer, that is, during the pressure application by the wearer based on the operation guide, the wearable device may detect a stability situation during the pressure application. If the stability situation does not satisfy a data collection requirement, the blood pressure measurement is ended.

If the acceleration of the wearable device is greater than the acceleration threshold, it may indicate that the wearable device is in a shaking state and the requirement is not satisfied, and the blood pressure measurement is ended. If the angular velocity of the wearable device of the wearable device is greater than the angular velocity threshold, it may indicate that the wearable device is in a skewed warping state and the requirement is not satisfied, and the blood pressure measurement is ended. If the acceleration of the wearable device is greater than the acceleration threshold, and the angular velocity of the wearable device of the wearable device is greater than the angular velocity threshold, it may indicate that the wearable device is in the state of shaking and skewed warping and the requirement is not satisfied, and the blood pressure measurement is ended. The acceleration of the wearable device may be obtained through an accelerometer, and the angular velocity of the wearable device may be obtained through a gyroscope, but this application is not limited thereto.

According to the blood pressure measurement method of a wearable device provided in this application, the state of the wearable device is detected during pressure application by the wearer. When the wearable device is in an unstable state, the blood pressure measurement is ended, which is advantageous in causing the blood pressure measurement to be performed in a stable situation and ensure accuracy of the blood pressure measurement.

With reference to the first aspect, in some implementations of the first aspect, the method further includes: displaying a first interface, where the first interface is configured to display a pressure curve, prompt information, and a waveform of the second PPG signal, the pressure curve is the operation guide, and the prompt information is configured for prompting a pressing duration of the wearer.

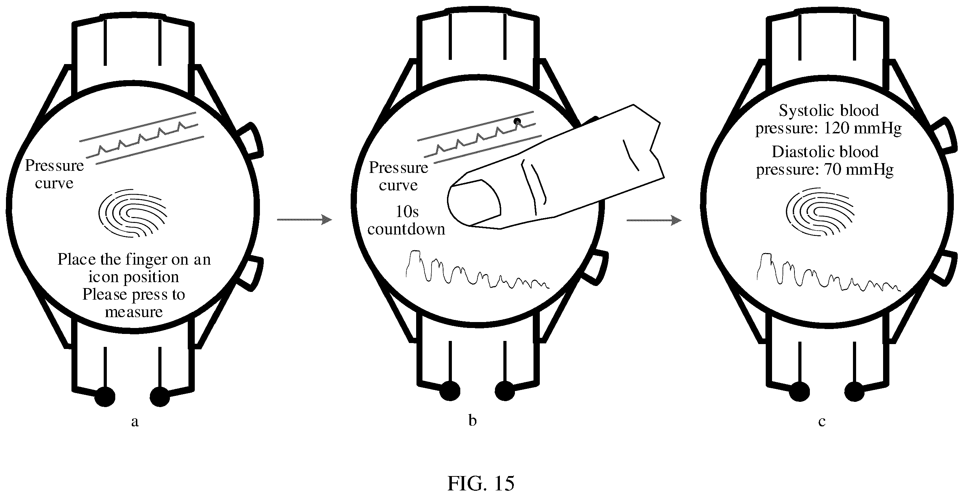

The first interface may also be referred to as an interface. This is not limited in embodiments of this application. The pressure curve is the operation guide. The wearer may apply the pressure to the wearable device based on the pressure curve, and the wearable device may indicate the user of the pressing duration through the prompt information to guide the user to continuously press for a period of time. The waveform of the second PPG signal may be the waveform of the second PPG signal collected by the wearable device. In a possible implementation, the first interface may be an interface b in FIG. 15 in embodiments.

According to the blood pressure measurement method of a wearable device provided in this application, the pressure curve, the prompt information, and the waveform of the PPG signal are displayed through the first interface. This is advantageous for the user to correctly apply the pressure to the wearable device based on the operation guide, may improve user experience, and is also advantageous in ensuring correct measurement of the blood pressure value.

With reference to the first aspect, in some implementations of the first aspect, before the emitting an optical signal through a first region of the display screen, the method further includes: determining whether the wearable device satisfies a blood pressure measurement condition; and the emitting an optical signal through a first region of a display screen includes: emitting the optical signal through the first region of the display screen when the blood pressure measurement condition is satisfied.

The blood pressure measurement condition may be a measurement condition in embodiments. When the blood pressure measurement condition is satisfied, the blood pressure of the wearer is measured. It may be understood that if the wearable device satisfies the blood pressure measurement condition, it may indicate that the blood pressure measurement may be effectively performed in a current state, and the blood pressure value has relatively high accuracy. If the wearable device does not satisfy the blood pressure measurement condition, it may indicate that the blood pressure measurement cannot be effectively performed in the current state, and the blood pressure value has relatively low accuracy.

The blood pressure measurement condition may include at least one of the wearable device being in a relatively static state, the wearable device being in good contact with the skin, and the PPG signal having good quality.

The measurement condition may include at least one of the wearable device being in a relatively static state, the wearable device being in good contact with the skin, and the wearable device being not skewed and warped.

The wearable device may obtain the acceleration of the wearable device through an accelerometer (accelerometer, ACC) to determine whether the wearable device is in the relatively static state. The wearable device may obtain the pressure value through the PT to determine whether the wearable device is in good contact with the skin. The wearable device may obtain angular velocity data through the gyroscope to determine whether the wearable device is skewed and warped, but this embodiment of this application is not limited thereto.

According to the blood pressure measurement method of a wearable device provided in this application, it is determined whether the blood pressure measurement condition is satisfied before the blood pressure measurement is performed. Measurement is performed on the basis of satisfying the blood pressure measurement condition, which is advantageous in improving the accuracy of the blood pressure value.

With reference to the first aspect, in some implementations of the first aspect, the measurement condition includes at least one of the following: the acceleration of the wearable device is less than or equal to the acceleration threshold, the pressure value is greater than or equal to a pressure threshold, or the angular velocity of the wearable device is less than or equal to the angular velocity threshold, where the acceleration is configured for representing a contact situation between the wearable device and the wearer, and the pressure value is configured for representing a tightness for wearing the wearable device.

The pressure value is obtained before the wearable device controls the first region of the display screen to emit light.

With reference to the first aspect, in some implementations of the first aspect, the display screen is supported by a screen bracket, the screen bracket includes an optical window, the PD is directly below the optical window, a preset distance is defined between the PD and the optical window, the PT is located at a bottom of the wearable device and is configured to collect a pressure value between the wearable device and a wearing part, and the wearing part is a contact part between the wearable device and the wearer.

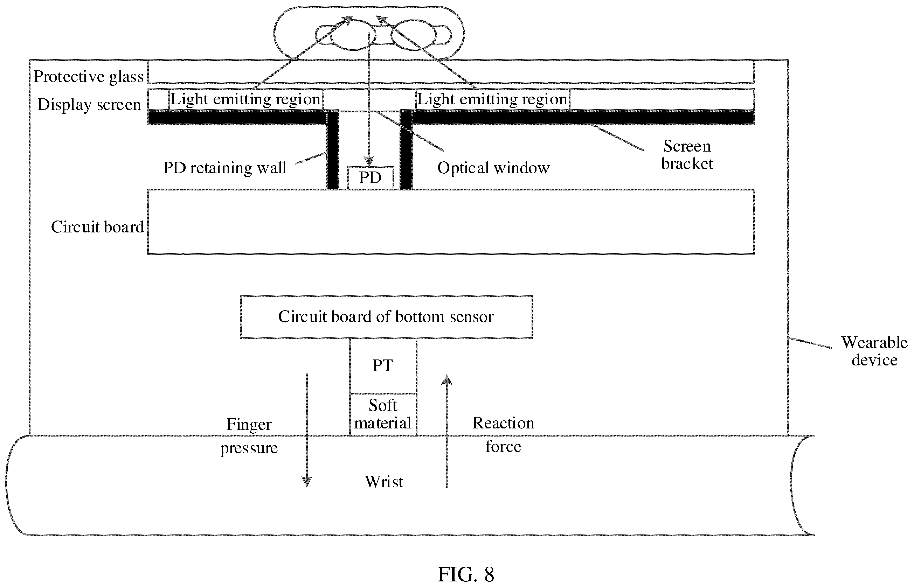

A structure of the wearable device may be shown in FIG. 8 in embodiments. The screen bracket is configured to support the display screen. The display screen includes the optical window for the PD to receive the optical signal. A specific distance is defined between the PD and the optical window, which may be referred to as the preset distance, but this application is not limited thereto. The PT is located at the bottom of the wearable device and may be integrated into a circuit board of a sensor. The PT is configured to collect the pressure value between the wearable device and the wearing part. In other words, the wearable device is pressed with a finger, so that a reaction force may be formed between the wearable device and the wearing part. The wearable device may calculate a finger pressure based on a relationship between the reaction force and an acting force (which may be the finger pressure). The wearable device may obtain the blood pressure value based on the finger pressure and a PPG signal of the finger.

In the blood pressure measurement method of a wearable device provided in this application, the PT and the PD are separately deployed, rather than in the form of an integrated module, and may be deployed in the wearable device in a scattered manner, which is flexible and may also save space.

According to a second aspect, a wearable device is provided. The wearable device includes a pressure transducer PT, a display screen, and a photo diode PD. The wearable device further includes a processing module and an obtaining module. The processing module is configured to: emit an optical signal through a first region of a display screen; convert, into a first PPG signal, the optical signal received by the PD; emit the optical signal through a second region of the display screen if the first PPG signal does not satisfy a signal quality requirement, where the second region and the first region are different in at least one of the following features: a position, luminance, an area size, or a shape on the display screen; and convert, into a second PPG signal, the optical signal received by the PD. The obtaining module is configured to obtain a pressure value collected by the PT when the second PPG signal satisfies the signal quality requirement. The processing module is further configured to determine a blood pressure value of a wearer based on the pressure value and the second PPG signal.

With reference to the second aspect, in some implementations of the second aspect, that the first PPG signal does not satisfy a signal quality requirement includes: a perfusion rate of the first PPG signal is less than or equal to a preset perfusion rate; and a distance between a projection region of the PD on the display screen and the second region is greater than a distance between the projection region of the PD on the display screen and the first region.

With reference to the second aspect, in some implementations of the second aspect, that the first PPG signal does not satisfy a signal quality requirement includes: an intensity of the first PPG signal is less than or equal to a preset intensity; and the luminance of the second region is greater than the luminance of the first region.

With reference to the second aspect, in some implementations of the second aspect, the processing module is further configured to: emit the optical signal through a third region of the display screen if the second PPG signal does not satisfy the signal quality requirement, where a distance between a projection region of the PD on the display screen and the third region is less than a distance between the projection region of the PD on the display screen and the first region; and convert, into a third PPG signal, the optical signal received by the PD. The obtaining module is further configured to obtain a pressure value collected by the PT if the third PPG signal satisfies the signal quality requirement. The processing module is further configured to determine the blood pressure value of the wearer based on the pressure value and the third PPG signal.

With reference to the second aspect, in some implementations of the second aspect, the shape of the second region is different from the shape of the first region.

With reference to the second aspect, in some implementations of the second aspect, the area size of the second region is different from the area size of the first region.

With reference to the second aspect, in some implementations of the second aspect, the pressure value includes a plurality of pressure values, the second PPG signal includes a plurality of second PPG signals, the plurality of pressure values are in a one-to-one correspondence with the plurality of second PPG signals, and a collection moment of each of the plurality of pressure values is the same as that of a corresponding one of the second PPG signals. The foregoing processing module is further configured to: extract a waveform feature of each of the plurality of second PPG signals; and determine, if a target second PPG signal whose waveform feature is less than or equal to a preset waveform feature exists in the plurality of second PPG signals, a pressure value corresponding to the target second PPG signal as a systolic blood pressure SBP in the blood pressure value of the wearer.

With reference to the second aspect, in some implementations of the second aspect, the pressure value includes a plurality of pressure values, the second PPG signal includes a plurality of second PPG signals, the plurality of pressure values are in a one-to-one correspondence with the plurality of second PPG signals, and the collection moment of each of the plurality of pressure values is the same as that of the corresponding second PPG signal. The foregoing processing module is further configured to: extract a waveform feature of each of the plurality of second PPG signals; input the plurality of pressure values and the waveform features of the plurality of second PPG signals into a neural network model, where the neural network model is trained based on a historical pressure value and a waveform feature of a historical PPG signal, and is configured to measure blood pressure; and determine output of the neural network model as a diastolic blood pressure DBP in the blood pressure value of the wearer.

With reference to the second aspect, in some implementations of the second aspect, the foregoing processing module is further configured to output an operation guide, where the operation guide is configured to guide the wearer to apply a pressure perpendicular to a contact surface between the wearable device and human skin to the wearable device in a preset pressing region, and the operation guide includes a pressure value required for the wearer to press.

With reference to the second aspect, in some implementations of the second aspect, the foregoing processing module is further configured to end, during the pressure application by the wearer, blood pressure measurement if the following condition is satisfied: an acceleration of the wearable device is greater than an acceleration threshold; and/or an angular velocity of the wearable device is greater than an angular velocity threshold.

With reference to the second aspect, in some implementations of the second aspect, the foregoing wearable device further includes a display module. The display module is configured to display a first interface, where the first interface is configured to display a pressure curve, prompt information, and a waveform of the second PPG signal, the pressure curve is the operation guide, and the prompt information is configured for prompting a pressing duration of the wearer.

With reference to the second aspect, in some implementations of the second aspect, the foregoing processing module is further configured to: determine whether the wearable device satisfies a blood pressure measurement condition; and emit the optical signal through the first region of the display screen when the blood pressure measurement condition is satisfied.

With reference to the second aspect, in some implementations of the second aspect, the measurement condition includes at least one of the following: the acceleration of the wearable device is less than or equal to the acceleration threshold, the pressure value is greater than or equal to a pressure threshold, or the angular velocity of the wearable device is less than or equal to the angular velocity threshold, where the acceleration is configured for representing a contact situation between the wearable device and the wearer, and the pressure value is configured for representing a tightness for wearing the wearable device.

With reference to the second aspect, in some implementations of the second aspect, the display screen is supported by a screen bracket, the screen bracket includes an optical window, the PD is directly below the optical window, a preset distance is defined between the PD and the optical window, the PT is located at a bottom of the wearable device and is configured to collect a pressure value between the wearable device and a wearing part, and the wearing part is a contact part between the wearable device and the wearer.

According to a third aspect, this application provides a wearable device, which may also be referred to as a blood pressure measurement apparatus, including a processor. The processor is coupled to a memory, and may be configured to execute an instruction in the memory, to implement the method according to any one of the possible implementations of the foregoing first aspect. Optionally, the wearable device further includes the memory. Optionally, the wearable device further includes a transceiver. The processor is coupled to the transceiver.

According to a fourth aspect, this application provides a processor, including an input circuit, an output circuit, and a processing circuit. The processing circuit is configured to receive a signal by using the input circuit and transmit a signal by using the output circuit, so that the processor performs the method according to any one of the possible implementations of the foregoing first aspect.

In a specific implementation process, the foregoing processor may be a chip, the input circuit may be an input pin, the output circuit may be an output pin, and the processing circuit may be a transistor, a gate circuit, a flip-flop, various logic circuits, and the like. An inputted signal received by the input circuit may be for example without limitation received and inputted by a receiver, a signal outputted by the output circuit may be for example without limitation outputted to and transmitted by a transmitter, and the input circuit and the output circuit may be a same circuit. The circuit is used as the input circuit and the output circuit at different moments. Specific implementations of the processor and various circuits are not limited in this application.

According to a fifth aspect, this application provides a processing apparatus, including a processor and a memory. The processor is configured to read an instruction stored in the memory, and may receive a signal by using a receiver and transmit a signal by using a transmitter, to perform the method according to any one of the possible implementations of the foregoing first aspect.

Optionally, one or more processors are provided, and one or more memories are provided.

Optionally, the memory may be integrated with the processor, or the memory is arranged separately from the processor.

In a specific implementation process, the memory may be a non-transitory (non-transitory) memory, for example, a read-only memory (read-only memory, ROM). The memory and the processor may be integrated on a same chip, or may be separately arranged on different chips. A type of the memory and a manner of arranging the memory and the processor are not limited in this application.

It is to be understood that a related data interaction process, for example, sending indication information, may be a process of outputting indication information from the processor, and receiving capability information may be a process of receiving input capability information by the processor. Specifically, data outputted by the processing may be outputted to the transmitter, and input data received by the processor may be from the receiver. The transmitter and the receiver may be collectively referred to as a transceiver.

The processing apparatus in the foregoing fifth aspect may be a chip. The processor may be implemented by hardware, or may be implemented by software. When the processor is implemented by hardware, the processor may be a logic circuit, an integrated circuit, and the like. When the processor is implemented by software, the processor may be a general-purpose processor, and is implemented by reading software code stored in a memory. The memory may be integrated into the processor, may be located outside the processor, or may exist independently.

According to a sixth aspect, this application provides a computer-readable storage medium. The computer-readable storage medium stores a computer program (which may also be referred to as code or an instruction). When the computer program is run on a computer, the computer is enabled to perform the method in any one of the possible implementations of the foregoing first aspect.

According to a seventh aspect, this application provides a computer program product. The computer program product includes a computer program (which may also be referred to as code or an instruction). When the computer program is run, the computer is enabled to perform the method in any one of the possible implementations of the foregoing first aspect.

### BRIEF DESCRIPTION OF DRAWINGS

FIG. 1 is a schematic diagram of a blood pressure measurement scene according to an embodiment of this application;
FIG. 2 is a schematic structural diagram of a communication system according to an embodiment of this application;
FIG. 3 is a schematic structural diagram of another communication system according to an embodiment of this application;
FIG. 4 is a schematic cross-sectional view of a hardware structure of a wearable device according to an embodiment of this application;
FIG. 5 is a top view of a wearable device according to an embodiment of this application;
FIG. 6 is a schematic cross-sectional view of a hardware structure of another wearable device according to an embodiment of this application;
FIG. 7 is a top view of another wearable device according to an embodiment of this application;
FIG. 8 is a schematic cross-sectional view of a hardware structure of still another wearable device according to an embodiment of this application;
FIG. 9 is a schematic flowchart of a blood pressure measurement method of a wearable device according to an embodiment of this application;
FIG. 10 is a schematic flowchart of a method for determining a measurement condition according to an embodiment of this application;
FIG. 11 is a schematic flowchart of a method for adjusting a light emitting region according to an embodiment of this application;
FIG. 12 is a schematic flowchart of a method for calculating a blood pressure value according to an embodiment of this application;
FIG. 13 is a schematic diagram of waveform curve changes of a PPG signal according to an embodiment of this application;
FIG. 14 is a schematic diagram of a waveform of a PPG signal according to an embodiment of this application;
FIG. 15 is a schematic diagram of a blood pressure measurement interface according to an embodiment of this application;
FIG. 16 is a schematic block diagram of a wearable device according to an embodiment of this application; and
FIG. 17 is a schematic block diagram of another wearable device according to an embodiment of this application.

### DESCRIPTION OF EMBODIMENTS

The following describes technical solutions of this application with reference to the accompanying drawings.

Currently, a plurality of blood pressure measurement methods exist on the market, and through research on various measurement methods in this application, it is found that accuracy of a blood pressure value is difficult to guarantee.

A common blood pressure measurement manner is obtaining a blood pressure value based on a PPG signal of a wrist. The waveform of the PPG signal may also be referred to as a pulse wave waveform, and includes important feature information of human blood pressure. In general, a main peak of the waveform of the PPG signal corresponds to a systolic blood pressure (that is, a high pressure in the blood pressure value), and a replay wave peak corresponds to a diastolic blood pressure (that is, a low pressure in the blood pressure value). A wearable device includes a PPG module. The PPG module contacts human skin to collect a PPG signal of a human body.

Through research on the method in this application, it is found that the method has a high requirement for a wearing tightness. If the human skin is in poor contact with the PPG module, the PPG signal is inaccurate, and then the blood pressure value is inaccurate.

Another common blood pressure measurement manner is obtaining a blood pressure value based on a PPG signal of a fingertip. The PPG signal of the fingertip is usually superior and is considered as an ideal position for measuring blood pressure. When the wearable device detects that a finger touches the PPG module, the PPG signal of the finger is collected through the PPG module, and the blood pressure value is obtained through analysis of the PPG signal.

According to the method, different users touch the PPG module with different strengths, which causes different waveform features of the PPG signal and different measurement conditions of different users and affects measurement accuracy of the users. In addition, the PPG module is deployed on a side surface of a watch frame of the wearable device, and a size of the PPG module affects appearance design of a wearable watch, causing a surface body to be thicker and unable to be thinned, and affecting user experience. Besides, a structure of the PPG module according to the method is different from that of the PPG module for measuring the wrist, which needs to be developed independently by a researcher and is not conducive to wide application.

To resolve a problem of different measurement conditions of different users, a pressure transducer PT is added to the PPG module of the wearable device to ensure that different users have a same measurement condition. However, in terms of a limited internal space of a wearable product, adding the PT causes greater difficulties and bottlenecks in stacked design.

Still another common blood pressure measurement manner is that a user applies a pressure to a wearable device through a fingertip, and the wearable device obtains a pressure of the fingertip through a PT, and emits an optical signal through an LED in a PPG module. The optical signal is absorbed and scattered by skin tissue of the fingertip to form a reflected signal. The reflected signal may be received by a PD in the PPG module and converted into a PPG signal. The wearable device may obtain the blood pressure value based on a waveform of the PPG signal of the fingertip.

The method has a problem that the blood pressure value obtained through measurement is inaccurate.

In view of this, embodiments of this application provide a blood pressure measurement method of a wearable device and a wearable device. The user is guided to touch and press a screen of the wearable device with the finger, a light emitting unit of the screen is used as a measurement light source for the blood pressure measurement, the optical signal reflected by the finger is received by using the photo diode (photo diode, PD), and the optical signal is converted into the PPG signal. The pressure value applied by the fingertip during the pressure application is obtained through the PT, and the blood pressure value is calculated based on the pressure value and the waveform feature of the PPG signal. According to the method, the systolic blood pressure (that is, a high pressure) of the blood pressure may be directly measured through the PT, and the diastolic blood pressure (that is, a low pressure) is calculated through the waveform feature presented by the PPG signal of the fingertip. Compared with the blood pressure value calculated through regression or another calculation method, accuracy of the blood pressure value is improved. In addition, the user may directly press the screen by using the finger. Compared with collection of the fingertip pressure by pressing the side surface of the watch frame, the stability is better, which is advantageous in ensuring signal quality of the PPG signal. In addition, according to the method provided in embodiments of this application, the light emitting unit of the screen is reused, and the measurement light source does not need to be separately deployed, which may save costs and reduce the size of the PPG module. Moreover, in embodiments of this application, at least one of a distance between the measurement light source and the PD, a shape of the measurement light source, distribution of the measurement light source, or a luminous intensity of the measurement light source may also be adjusted, to improve the signal quality of the PPG signal, which is advantageous in improving accuracy of the blood pressure value.

To have a better understanding of embodiments of this application, an embodiment of this application provides a blood pressure measurement scene.

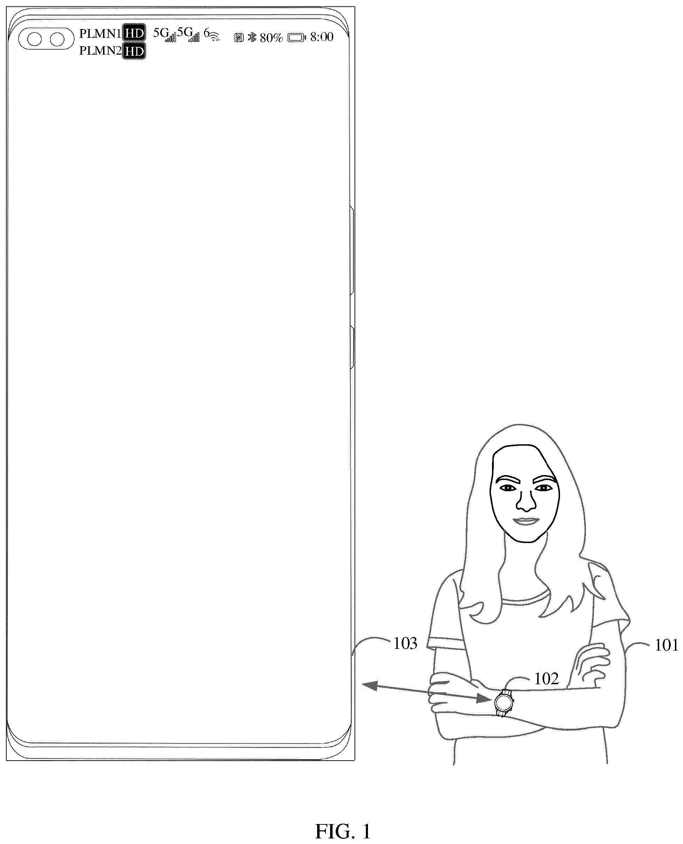

For example, FIG. 1 is a schematic diagram of a blood pressure measurement scene. As shown in FIG. 1, the blood pressure measurement scene includes a user 101, a wearable device 102, and a terminal device 103. The user 101 wears the wearable device 102 on a wrist. The wearable device 102 may support a function of blood pressure measurement. The terminal device 103 may perform data communication with the wearable device 102.

In the scene, this embodiment of this application provides two blood pressure measurement methods of a wearable device.

For a first blood pressure measurement method of a wearable device, the wearable device 102 may include a PT, a PPG module, and an accelerometer (accelerometer, ACC). The wearable device 102 may obtain an acceleration of the wearable device 102 through the ACC, and may determine, based on the acceleration, whether the wearable device 102 is in a relatively static state. When the wearable device 102 is in the relatively static state, the user 101 is guided to touch and press a screen of the wearable device 102 by using a finger, a light emitting unit of the screen is used as a measurement light source for blood pressure measurement, an optical signal reflected by the finger is received by using a photo diode (photo diode, PD), and the optical signal is converted into a PPG signal. A pressure value during the pressure application by the user 101 is obtained through the PT, and a blood pressure value is calculated based on the pressure value and the PPG signal. The wearable device 102 may transmit a measured blood pressure value to the terminal device 103. The terminal device 103 may save and display the blood pressure value.

For a second blood pressure measurement method of a wearable device, the wearable device 102 may obtain an acceleration of the wearable device 102 through the ACC, and determines whether the wearable device 102 is in a relatively static state. When the wearable device 102 is in the relatively static state, the user 101 is guided to touch and press a screen of the wearable device 102 by using a finger, a light emitting unit of the screen is used as a measurement light source for blood pressure measurement, an optical signal reflected by the finger is received by using a PD, and the optical signal is converted into a PPG signal. A pressure value during the pressure application by the user 101 is obtained through a PT, and the pressure value and the PPG signal are transmitted to the terminal device 103. The terminal device 103 may calculate a blood pressure value based on the pressure value and the PPG signal. The terminal device 103 may save and display the blood pressure value. The terminal device 103 may also transmit the blood pressure value to the wearable device 102. The wearable device 102 may save and display the blood pressure value.

According to the blood pressure measurement method of a wearable device, computing power of the wearable device 102 may be saved, and power consumption of the wearable device 102 may be reduced.

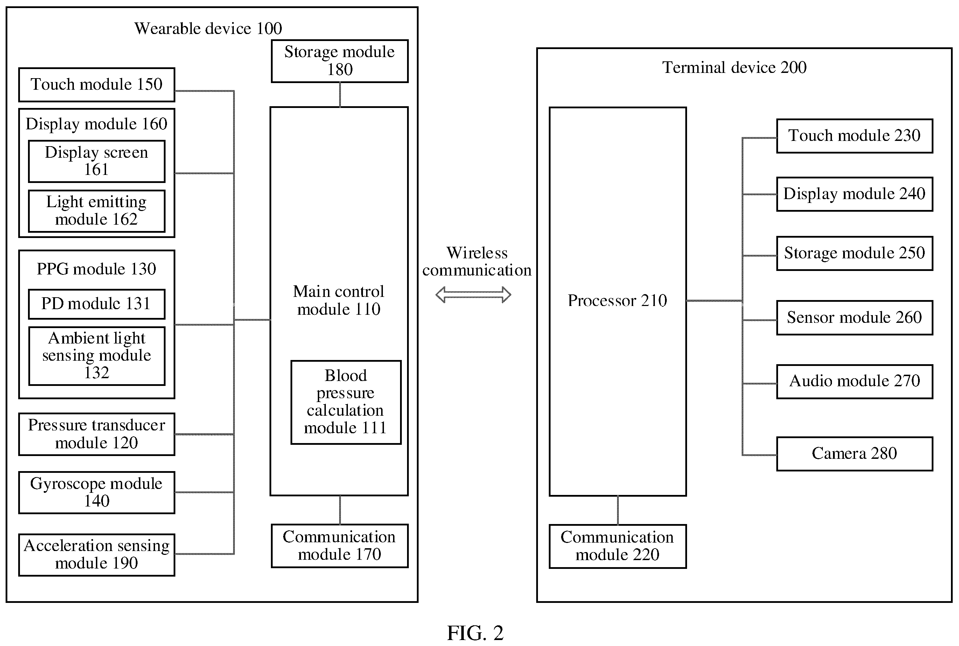

To have a better understanding of embodiments of this application, the following describes a structure of a communication system in embodiments of this application. For example, FIG. 2 is a schematic structural diagram of a communication system according to an embodiment of this application.

As shown in FIG. 2, the communication system may include a wearable device 100 and a terminal device 200.

The wearable device 100 may be a smartwatch, a smart bracelet, or the like. This is not limited in embodiments of this application. A specific technology and a specific device form used by the wearable device are not limited in embodiments of this application.

The terminal device 200 may be a device such as a mobile phone or a tablet computer. This is not limited in embodiments of this application. A specific technology and a specific device form used by the terminal device are not limited in embodiments of this application.

As shown in FIG. 2, the wearable device 100 and the terminal device 200 may transmit data information to each other through a communication module.

The wearable device 100 may include a main control module 110, a pressure transducer module 120, a PPG module 130, a gyroscope module 140, a touch module 150, a display module 160, a communication module 170, a storage module 180, and an acceleration sensing module 190. The modules may be connected through a bus or in another manner. This is not limited in embodiments of this application.

It may be understood that a schematic structure of this embodiment of the present invention does not constitute a specific limitation on the wearable device 100. In some other embodiments of this application, the wearable device 100 may also include more or fewer components than shown in the figure, or some merged components, or some split components, or different component arrangements. The components shown in the figure may be implemented by hardware, software, or a combination of software and hardware.

The main control module 110 may include a blood pressure calculation module 111. The blood pressure calculation module 111 may be configured to calculate a blood pressure value. The main control module 110 may also include one or more processors for supporting other functions of the wearable device 100.

The pressure transducer module 120 may be configured to collect pressure data of the wearable device. In a possible implementation, the pressure transducer module 120 may be configured to collect a pressure value of the wearable device pressed by a finger.

The PPG module 130 may include a photo diode (photo diode, PD) module 131 and an ambient light sensing module 132. The PD module 131 may include one or more PDs. This is not limited in embodiments of this application. The PD may be configured to receive an optical signal and process the optical signal into an electrical signal. For example, in a scene of measuring blood pressure, the PD may receive an optical signal reflected by fingertip tissue and process the signal into an electrical signal. The ambient light sensing module 132 may be an ambient light sensor, and may be configured to sense luminance of ambient light. For example, in a scene of measuring the blood pressure, the ambient light sensor may detect luminance of a measurement light source.

The gyroscope module 140 may be a gyroscope sensor, and may be configured to obtain angular velocity data of the wearable device 100.

The touch module 150 may be a touch sensor. The touch sensor may also be referred to as a "touch control device".

The display module 160 may include a display screen 161 and a light emitting module 162. The light emitting module 162 may emit light for causing the display screen 161 to display content. In the scene of measuring the blood pressure, the light emitting module 162 may also be used as the measurement light source. The touch sensor may be arranged in the display screen 161, and the touch sensor and the display screen 161 form a touch screen, which may also be referred to as a "touch control screen". The touch sensor may be configured to detect a touch operation performed on or near the touch sensor. The display screen 161 may be configured to display an image, a video, a control, text information, and the like.

The communication module 170 may include a bluetooth communication module and a WLAN communication module. In some embodiments, the WLAN communication module may be integrated with another communication module (for example, the bluetooth communication module). The WLAN communication module and/or the bluetooth module may transmit a signal to detect and scan a device near the wearable device 100, so that the wearable device 100 may discover a nearby device such as the terminal device 200 by using one or more wireless communication technologies such as bluetooth or WLAN. The wearable device 100 may establish a wireless communication connection with the terminal device 200 by using one or more wireless communication technologies such as WLAN and bluetooth, and perform data transmission and data receiving based on the wireless communication connection. The wearable device 100 may transmit data to the terminal device 200 based on one or more wireless communication technologies of the bluetooth communication module or the WLAN communication module. The wearable device 100 may also obtain, through the wireless communication connection, a data instruction transmitted by the terminal device 200.

The storage module 180 is coupled to the main control module 110, and is configured to store various software programs and/or a plurality of sets of instructions. In a specific implementation, the storage module 180 may include a volatile memory (volatile memory), for example, a random access memory (random access memory, RAM), or may include a non-volatile memory (non-volatile memory), for example, a ROM, a flash memory (flash memory), a hard disk drive (hard disk drive, HDD), or a solid state drive (solid state drive, SSD). The storage module 180 may also include a combination of the foregoing types of memories. The storage module 180 may store some program code, so that the main control module 110 invokes the program code stored in the storage module 180, to implement the method of embodiments of this application.

The acceleration sensing module 190 may be an accelerometer, and may be configured to detect magnitudes of accelerations of the wearable device 100 in all directions (generally on three axes).

The terminal device 200 may include a processor 210, a communication module 220, a touch module 230, a display module 240, a storage module 250, a sensor module 260, an audio module 270, and a camera 280. The modules may be connected through a bus or in another manner. This is not limited in embodiments of this application.

It may be understood that the schematic structure in this embodiment of the present invention does not constitute a specific limitation on the terminal device 200. In some other embodiments of this application, the terminal device 200 may also include more or fewer components than shown in the figure, or some merged components, or some split components, or different component arrangements. The components shown in the figure may be implemented by hardware, software, or a combination of software and hardware.

The processor 210 may include one or more processing units. Different processing units may be independent devices, or may be integrated into one or more processors. A memory may also be arranged in the processor 210 to store an instruction and data.

The communication module 220 may include a mobile communication module and a wireless communication module. The mobile communication module may provide a solution to the wireless communication technology including the 2^{nd} generation (2^{nd} generation, 2G) communication technology/3^{rd} generation (3^{rd} generation, 3G) communication technology/4^{th} generation (4^{th} generation, 4G) communication technology/5^{th} generation (5^{th} generation, 5G) communication technology applied to the terminal device 200. The mobile communication module may include at least one filter, a switch, a power amplifier, a low noise amplifier (low noise amplifier, LNA), and the like. The wireless communication module may provide solutions to wireless communication technologies including a wireless local area network (wireless local area network, WLAN) (such as a wireless fidelity (wireless fidelity, Wi-Fi) network), bluetooth (bluetooth, BT), a global navigation satellite system (global navigation satellite system, GNSS), frequency modulation (frequency modulation, FM), and the like to be applied to the terminal device 200.

The touch module 230 may be a touch sensor. The touch sensor may also be referred to as a "touch control device".

The display module 240 may be a display screen. The touch sensor may be arranged in the display screen, and the touch sensor and the display screen form a touch screen, which may also be referred to as a "touch control screen". The touch sensor may be configured to detect a touch operation performed on or near the touch sensor. The display screen may be configured to display an image, a video, a control, text information, and the like.

The storage module 250 may include an internal memory or an external memory. The external memory may be configured to connect to an external memory card, for example, a Micro SD card, to expand a storage capacity of the terminal device 200. The external memory card communicates with the processor 210 through the external memory, to implement a data storage function. For example, files such as music and a video are saved in the external memory card. The internal memory may be configured to store computer-executable program code. The executable program code includes an instruction. The internal memory may include a program storage area and a data storage area.

The sensor module 260 may include sensors such as a pressure transducer, a gyroscope sensor, a barometric pressure transducer, a magnetic sensor, an accelerometer, a distance sensor, an optical proximity sensor, a fingerprint sensor, a temperature sensor, an ambient light sensor, a bone conduction sensor, and the like. The pressure transducer is configured to sense a pressure signal, and may convert the pressure signal into an electrical signal. In some embodiments, the pressure transducer may be arranged in the display screen. The gyroscope sensor may be configured to determine a movement posture of the terminal device 200. The barometric pressure transducer is configured to measure an air pressure. The magnetic sensor includes a Hall sensor. The accelerometer may detect magnitudes of accelerations of the terminal device 200 in all directions (generally on three axes). The distance sensor is configured to measure a distance. The optical proximity sensor may include, for example, a light emitting diode (LED) and an optical detector, for example, a photo diode. The ambient light sensor is configured to sense luminance of ambient light. The fingerprint sensor is configured to collect a fingerprint. The temperature sensor is configured to detect a temperature. The bone conduction sensor may obtain a vibration signal.

The audio module 270 is configured to convert digital audio information into an analog audio signal output, and is also configured to convert an analog audio input into a digital audio signal.

The camera 280 may be configured to capture a static image or a video. In some embodiments, the terminal device 200 may include 1 or N cameras, where N is a positive integer greater than 1.

It is to be noted that the wearable device 100 shown in FIG. 2 provided in this embodiment of this application may perform the foregoing first blood pressure measurement method. In other words, the wearable device 100 determines an operating state of the wearable device 100 by using the data collected by the acceleration sensing module 190, and determines the blood pressure value based on data collected by the pressure transducer 120 and the PPG module 130 through the blood pressure calculation module 111. The wearable device 100 may store the blood pressure value through the storage module 180, and may display the blood pressure value through the display module 160. The wearable device 100 may also transmit the blood pressure value to the terminal device 200 through the communication module 170.

The terminal device 200 may receive the blood pressure value through the communication module 220, may store the blood pressure value into the storage module 250, and may also display the blood pressure value through the display module 240.

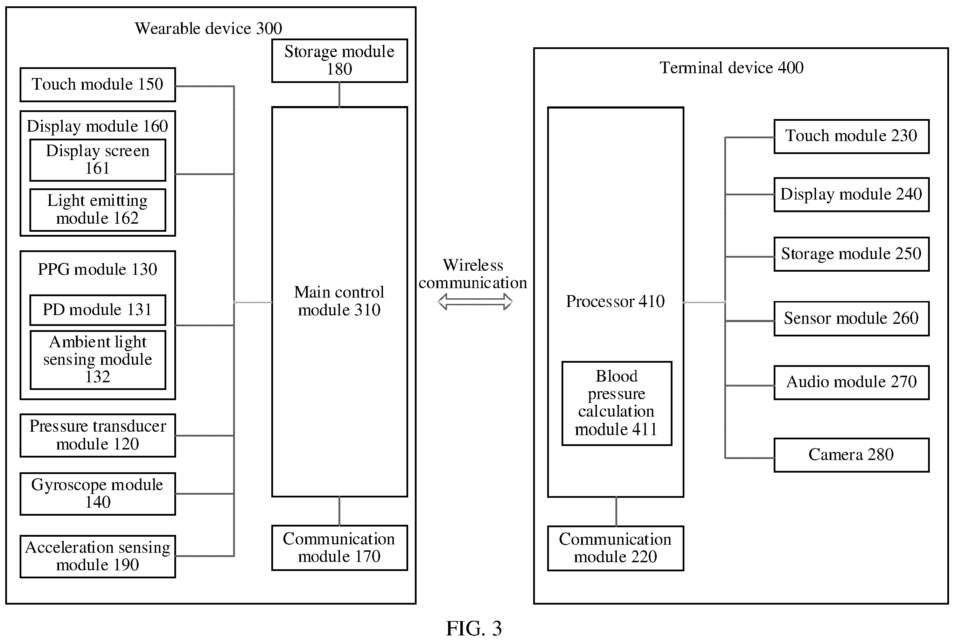

An embodiment of this application further provides a schematic structural diagram of another communication system. For example, FIG. 3 is a schematic structural diagram of another communication system according to an embodiment of this application. As shown in FIG. 3, the communication system may include a wearable device 300 and a terminal device 400.

The wearable device 300 may be a smartwatch, a smart bracelet, or the like. This is not limited in embodiments of this application. A specific technology and a specific device form used by the wearable device are not limited in embodiments of this application.

The terminal device 400 may be a device such as a mobile phone or a tablet computer. This is not limited in embodiments of this application. A specific technology and a specific device form used by the terminal device 400 are not limited in embodiments of this application.

As shown in FIG. 3, the wearable device 300 and the terminal device 400 may transmit data information to each other through a communication module.

The wearable device 300 may include a main control module 310, a pressure transducer module 120, a PPG module 130, a gyroscope module 140, a touch module 150, a display module 160, a communication module 170, a storage module 180, and an acceleration sensing module 190. The modules may be connected through a bus or in another manner. This is not limited in embodiments of this application.

The main control module 310 may include one or more processors for supporting functions of the wearable device 300. Other modules are all the same as above, and details are not described herein again.

Compared with the foregoing wearable device 100, the wearable device 300 has a different main control module. The main control module 310 in the wearable device 300 is not provided with a blood pressure calculation module 111. In other words, the wearable device 300 cannot calculate the blood pressure value, but may collect data and transmit the data communication module 170 to the terminal device 400. For example, the wearable device 300 collects the data through the pressure transducer 120 and the PPG module 130, and transmits the data to the terminal device 400 through the communication module 170. The wearable device 300 may receive, through the communication module 170, the blood pressure value transmitted by the terminal device 400, store the blood pressure value through the storage module 180, and display the blood pressure value through the display module 160.

The terminal device 400 may include a processor 410, a communication module 220, a touch module 230, a display module 240, a storage module 250, a sensor module 260, an audio module 270, and a camera 280. The modules may be connected through a bus or in another manner. This is not limited in embodiments of this application.

The processor 410 includes a blood pressure calculation module 211. The blood pressure calculation module 211 may be configured to calculate the blood pressure value. The processor 410 may include one or more processing units. Different processing units may be independent devices, or may be integrated into one or more processors. A memory may also be arranged in the processor 410 to store an instruction and data. Other modules are all the same as above, and details are not described herein again.

Compared with the foregoing terminal device 200, the processor 410 of the terminal device 400 includes a blood pressure calculation module 411. In other words, the terminal device 400 may calculate the blood pressure value. Specifically, the terminal device 400 may receive, through the communication module 220, the data transmitted by the wearable device 300, and obtain the blood pressure value based on the data through the blood pressure calculation module 411, may store the blood pressure value into the storage module 250, and may also display the blood pressure value through the display module 240. The terminal device 400 may also transmit the blood pressure value to the wearable device 300 through the communication module 220.

To have a better understanding of embodiments of this application, a schematic diagram of a hardware structure of the wearable device is described in detail in this embodiment of this application.

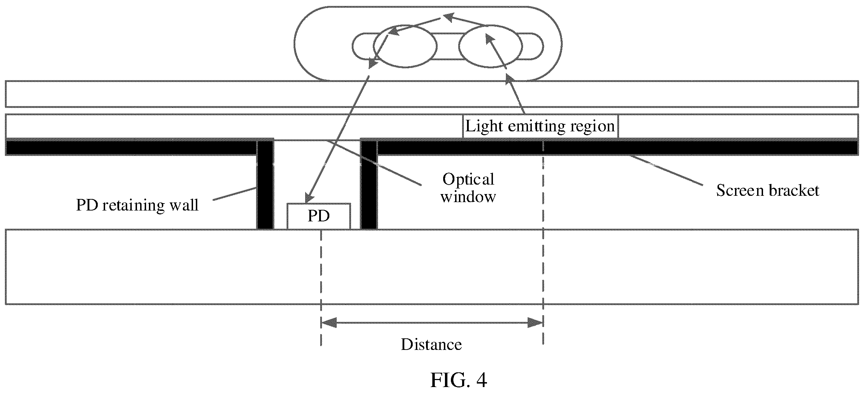

For example, FIG. 4 is a schematic cross-sectional view showing a hardware structure of a wearable device. The structure shown in FIG. 4 may be applicable to the foregoing wearable device 102, the wearable device 100, and the wearable device 300, but this embodiment of this application is not limited thereto.

As shown in FIG. 4, a display screen is used as a measurement light source, and protective glass is arranged above the screen to protect the display screen. The display screen is supported by a screen bracket. The screen bracket is made of an opaque material. When a light emitting region emits an optical signal, a light ray emitted downward by the light emitting region is blocked by the bracket and cannot be transmitted. The screen bracket is structurally designed with a light passing window (or referred to as an optical window). A PD is below the light passing window and above a circuit board, and PD retaining walls are provided on left and right sides of the PD. Each of the PD retaining walls may be configured to reflect an optical signal and improve an intensity of the optical signal received by the PD. The light emitting region and the PD are staggered by a specific distance in a horizontal direction, which is advantageous in increasing a light path after the light ray propagates in finger skin, increasing a perfusion rate of the optical signal, and preventing light directly reflected by a finger surface from being received by the PD. The distance between the light emitting region and the PD in the horizontal direction is variable, and the specific distance is not limited in embodiments of this application. It may be understood that a position of the PD is fixed, and the wearable device may adjust a position of the light emitting region of the screen.

The optical signal emitted by the light emitting region of the display screen may be a tri-color signal that integrates red, green, and blue, or a monochromatic optical signal, for example, red light, green light, blue light, or yellow light. This is not limited in embodiments of this application.

The optical signal emitted by the light emitting region of the display screen passes through the protective glass and is incident on skin tissue of the finger. The optical signal is absorbed and scattered by the skin tissue of the finger to form a reflected signal. The reflected signal passes through the light passing window and is received by the PD, to form a PPG signal. Only the light emitting region of the display screen emits light, and the rest does not emit light, which may avoid serious interference to the PPG signal as a result of the reflected optical signal that does not enter the skin of the finger being received by the PD.

A shape of the foregoing light emitting region may be a circle, an ellipse, a strip, a square, a triangle, a ring, an arc, or the like. The shape of the light emitting region depends on distribution of illuminated positions of light emitting units of the screen. The shape and an area size of the light emitting region are not limited in embodiments of this application.

In a possible implementation, the shape of the light emitting region may be the circle.

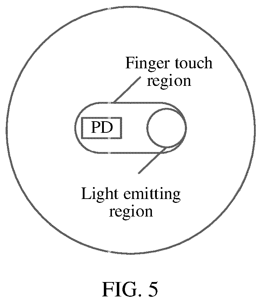

For example, FIG. 5 is a top view of a wearable device. As shown in FIG. 5, a shape of a light-emitting region is a circle. A finger touch region, that is, a region where a finger presses, may cover a light emitting region of a screen and a PD.

It may be understood that if the shape of the light emitting region is a circle, a cross-section of a hardware structure of the wearable device may be shown in FIG. 4 above.

In another possible implementation, the shape of the light emitting region may be a ring. The ring-shaped light emitting region is more advantageous in causing an optical signal emitted by the light emitting region to enter skin of the finger, so that the PD may receive more optical signals absorbed and scattered by internal tissue of the finger.

When the shape of the light emitting region is ring-shaped, the cross-section of the hardware structure of the wearable device may be displayed as the light emitting region on two sides of the PD.

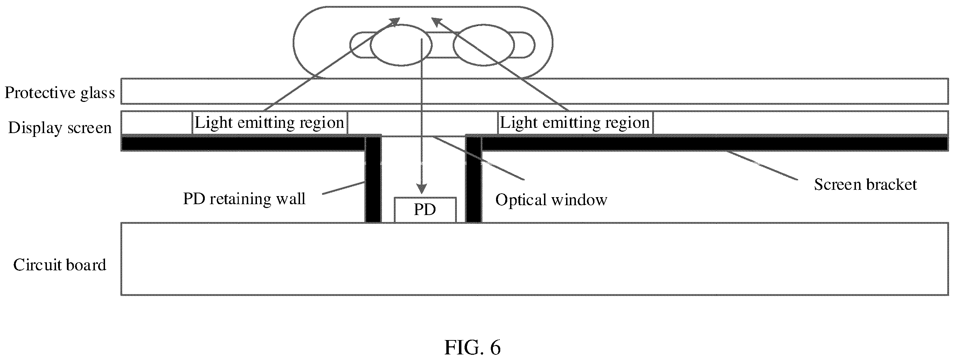

For example, FIG. 6 is a schematic cross-sectional view showing a hardware structure of a wearable device. As shown in FIG. 6, light emitting regions are on two sides of a PD, and the rest is the same as that shown in FIG. 4 above. Details are not described herein again in embodiments of this application.

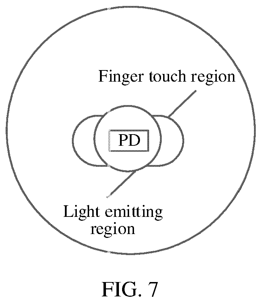

FIG. 7 is a top view of a wearable device. As shown in FIG. 7, a shape of a light emitting region may be a ring. A PD is at a central position of the ring and is at a central position of a finger touch region.

The wearable device provided in this embodiment of this application may also include a PT. The PT may be deployed in a bottom sensor of the wearable device. The PT may be configured to detect a direct pressure value of a finger touching a screen during blood pressure measurement, and return a pressure value to the wearable device. When a pressing pressure of the finger is different, a PPG waveform changes. The PPG waveform includes important feature information of human blood pressure. A great relationship exists between a shape of the PPG waveform and the pressure value on skin. During the blood pressure measurement, measurement accuracy may be further improved based on an actual finger pressure value.

For example, FIG. 8 is a schematic cross-sectional view showing a hardware structure of a wearable device. As shown in FIG. 8, a PT is deployed under a circuit board of a bottom sensor. The PT contacts a soft material and matches a housing of the wearable device in shape. A structure of another part is the same as that shown in FIG. 6 above. Details are not described herein again in embodiments of this application.

When a finger presses the wearable device, a reaction force formed on a bottom of a wearable product after a wearing part (such as wrist skin) of a user is pressed is transferred to the PT through the soft material. The PT may return the pressure value to the wearable device. The soft material is not limited to a material such as flexible resin, rubber, or silicone. Materials that can effectively transfer force are all within the scope of the present invention.

Optionally, in this embodiment, the shell of the wearable device may be designed to be arc-shaped. Since the PT is placed in the middle of the wearable device and is located at a center of a protrusion, the pressure value can be effectively transferred to the PT after the wearable device contacts the wrist skin to generate a pressure. When a rear housing of the wearable device is designed as a non-arc-shaped plane, the soft material used for filling may appropriately exceed the rear housing of the wearable device. When the wearable device contacts human skin, the pressure can be effectively detected.

The structure of the wearable device provided in embodiments of this application is described in detail above, and the method provided in embodiments of this application is to be described in detail below.

It is to be noted that the method provided in embodiments of this application may be used for measuring not only the blood pressure, but also a heart rate, blood oxygen, and blood glucose, so as to improve accuracy of a measurement result.

Before the technical solutions of embodiments of this application are described, the following description is first given.

First, in embodiments of this application, terms such as "first" and "second" are used to distinguish between same or similar items with basically same functions and roles. A person skilled in the art may understand that the terms such as "first" and "second" do not limit a quantity or an execution order, and the terms such as "first" and "second" are not limited to be definitely different.

Second, in this application, a term such as "example" or "for example" is used to represent giving an example, an illustration, or a description. Any embodiment or design scheme described by using "example" or "for example" in this application is not to be explained as being preferred or advantageous over another embodiment or design scheme. Exactly, use of the term such as "example" or "for example" is intended to present a related concept in a specific manner.

Third, the term "at least one" means one or more, and "a plurality of" means two or more. The term "and/or" is used for describing an association relationship between associated objects and representing that three relationships may exist. For example, A and/or B may represent the following three cases: only A exists, both A and B exist, and only B exists, where A and B may be singular or plural. The character "/" generally indicates an "or" relationship between a preceding associated object and a latter associated object. "At least one of the following items (pieces)" or a similar expression thereof refers to any combination of these items, including any combination of singular items (pieces) or plural items (pieces). For example, at least one item (piece) of a, b, and c may represent: a, or b, or c, or a and b, or a and c, or b and c, or a, b, and c, where a, b, and c may be singular or plural.

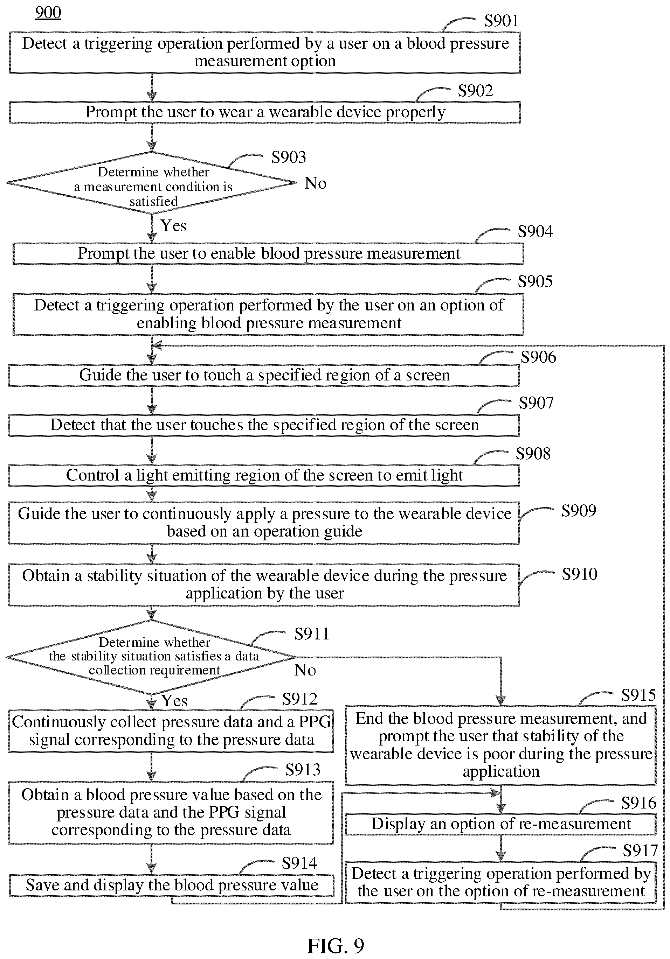

FIG. 9 is a schematic flowchart showing a blood pressure measurement method 900 of a wearable device. The method may be applicable to the scene shown in FIG. 1, but this embodiment of this application is not limited thereto. The method 900 may be performed by the wearable device, for example, the wearable device 102 shown in FIG. 1 above. A hardware structure diagram of the wearable device may be shown in FIG. 2, but this embodiment of this application is not limited thereto.

As shown in FIG. 9, the method 900 may include the following steps.

S901: Detect a triggering operation performed by a user on a blood pressure measurement option.

The wearable device may display the blood pressure measurement option through a human-machine interaction interface, and the user may click the blood pressure measurement option to enter a blood pressure measurement interface. When the user clicks the blood pressure measurement option, the wearable device may detect the triggering operation performed by the user on the blood pressure measurement option.

S902: Prompt, in response to the triggering operation performed by the user on the blood pressure measurement option, the user to wear the wearable device properly.

The wearable device may prompt, by using sound and/or texts, the user to wear the wearable device properly. The prompt content may be "please wear the device straight and tightly". If the wearable device provides a prompt through the texts, the prompt content may be displayed on an interface. If the wearable device provides a prompt through the sound, the prompt content may be played through a speaker. If the wearable device provides a prompt through the sound and the texts, the prompt content may be played through the speaker and displayed through an interface.

The wearable device prompts the user to wear the wearable device properly before a test, which is advantageous in causing the wearable device to have good contact with skin, and advantageous in improving quality of a collected PPG signal and then improving accuracy of blood pressure measurement.

S903: Determine whether a measurement condition is satisfied.

The wearable device determines whether the measurement condition is satisfied. In other words, the wearable device determines whether the blood pressure measurement may be performed in this case. If the measurement condition is satisfied, it may indicate that the blood pressure measurement may be effectively performed in a current state, and a blood pressure value has relatively high accuracy. If the measurement condition is not satisfied, it may indicate that the blood pressure measurement cannot be effectively performed in the current state, and the blood pressure value has relatively low accuracy. The measurement condition may also be referred to as a blood pressure measurement condition. This is not limited in embodiments of this application.

The measurement condition may include at least one of the wearable device being in a relatively static state, the wearable device being in good contact with the skin, and the wearable device being not skewed and warped.

The wearable device may obtain an acceleration of the wearable device through an ACC to determine whether the wearable device is in the relatively static state. The wearable device may obtain a pressure value through a PT to determine whether the wearable device is in good contact with the skin. The wearable device may obtain angular velocity data through the gyroscope to determine whether the wearable device is skewed and warped, but this embodiment of this application is not limited thereto.

S904: Prompt the user to enable blood pressure measurement if the measurement condition is satisfied.

The wearable device may prompt, by using sound and/or texts, the user to wear the wearable device properly. A prompt content may be "please start the blood pressure measurement". If the wearable device provides a prompt through the texts, the prompt content may be displayed on an interface. If the wearable device provides a prompt through the sound, the prompt content may be played through a speaker. If the wearable device provides a prompt through the sound and the texts, the prompt content may be played through the speaker and displayed through an interface.

S905: Detect a triggering operation performed by a user on an option of enabling blood pressure measurement.

The option of enabling blood pressure measurement may also be referred to as an option of starting blood pressure measurement. This is not limited in embodiments of this application.

The wearable device may enable the blood pressure measurement option through the human-machine interaction interface, and the user may click the option of enabling blood pressure measurement to enable blood pressure measurement. When the user clicks the option of enabling blood pressure measurement, the wearable device may perform the blood pressure measurement.

S906: Guide, in response to the triggering operation performed by the user on the option of enabling blood pressure measurement, the user to touch a specified region of a screen.

The wearable device may guide the user to touch the specified region of the screen through at least one prompt in the form of the texts, the sound, a picture, or an icon.

For example, the wearable device may display a fingerprint in the specified region of the screen and guide the user to touch a fingerprint region. The wearable device may guide the user to touch the fingerprint region through the texts. The text content may be "please place the finger in the fingerprint display region". The wearable device may also guide the user to touch the fingerprint region through the sound. The sound content may be "please place the finger in the fingerprint display region".

The specified region of the screen may be a finger touch region shown in FIG. 5 or FIG. 7 described above, but this embodiment of this application is not limited thereto.

S907: Detect an operation of touching the specified region of the screen by the user.

When the user is guided to touch the specified region of the screen, the wearable device may detect the operation of touching the specified region of the screen by the user.

S908: Control, in response to the operation of touching the specified region of the screen by the user, a light emitting region of the screen to emit light.

The wearable device controls the light emitting region of the screen to emit light, that is, controls the light emitting region of the screen to emit an optical signal. The light emitting region of the screen may also be referred to as a first region. This is not limited in embodiments of this application.

The light emitting region of the screen may be the light emitting region shown in FIG. 5 or FIG. 7 described above, but this embodiment of this application is not limited thereto.

S909: Guide the user to continuously apply a pressure to the wearable device based on an operation guide.

The wearable device may guide the user to continuously apply a pressure to the wearable device through texts, sound, an image, or a color. In other words, the operation guide may be the texts, the sound, the image, or the color. This is not limited in embodiments of this application. In a possible implementation, the operation guide may be "please increase the pressing pressure". The wearable device may guide the user through the sound and/or the texts. In another possible implementation, the wearable device displays a pressure curve through the human-machine interaction interface, and guides the user to continuously apply the pressure to the wearable device based on the pressure curve.

The wearable device may guide the user to continuously apply the pressure to the screen of the wearable device over a period of time based on the operation guide. The continuously applied pressure is perpendicular to the screen. A duration for the continuous pressure application is not limited is not limited in embodiments of this application. In a possible implementation, the duration for the continuous pressure application may be 30 seconds.

It is to be noted that the process of continuous pressure application is a process of increasing the pressure. In this embodiment of this application, blood vessels in the skin in contact with the wearable device are squeezed through the continuous pressure application by the user, so as to measure the blood pressure.

S910: Obtain a stability situation of the wearable device during the pressure application by the user.

On one hand, to prevent the wearable device in a shaking state during the pressure application from affecting collection of data and accuracy of the blood pressure value, the wearable device obtains the stability situation of the wearable device during the pressure application by the user.

For example, the wearable device may obtain the acceleration of the wearable device through the ACC to determine whether the wearable device is in the shaking state.

On the other hand, to prevent the wearable device from being skewed or warped and affecting collection of data and accuracy of the blood pressure value as a result of uneven pressure application by the user during the pressure application, the wearable device obtains the stability situation of the wearable device during the pressure application by the user.

For example, the wearable device may obtain an angular velocity of the wearable device through a gyroscope to determine whether the wearable device is in a skewed or warped state.

S911: Determine whether the stability situation satisfies a data collection requirement.

During the pressure application, if the wearable device is in the shaking state or in the skewed or warped state, the pressure data and the PPG signal are inaccurate, and the collection of the data is affected. If the wearable device is in a non-shaking state or in a non-skewed or non-warped state, the pressure data and the PPG signal are relatively accurate, and data collection may be performed. In other words, the data collection requirement is satisfied.

The wearable device determines whether the stability situation satisfies the data collection requirement. In other words, the wearable device determines whether the wearable device is in the shaking state or in the skewed or warped state during the pressure application.

If the stability situation of the wearable device satisfies the data collection requirement during the pressure application by the user, the data collection may be performed, that is, S912 is performed. If the stability situation of the wearable device does not satisfy the data collection requirement during the pressure application by the user, the blood pressure measurement may be ended, that is, S915 is performed.

S912: Continuously collect the pressure data and the PPG signal corresponding to the pressure data if the stability situation satisfies the data collection requirement.

If the stability situation satisfies the data collection requirement, the wearable device may continuously collect the pressure data and the PPG signal corresponding to the pressure data. In other words, during the pressure application by the user, the wearable device may collect a plurality of pieces of pressure data and simultaneously collect a PPG signal corresponding to each piece of pressure data. n pieces of pressure data may be collected, where n is an integer greater than or equal to 2.

Optionally, the wearable device may display a waveform of the PPG signal in real time on a display interface.

S913: Obtain the blood pressure value based on the pressure data and the PPG signal corresponding to the pressure data.

During the pressure application by the user, the pressure increases as the pressure application process progresses. Different pressure values correspond to different PPG signals. The wearable device may obtain the blood pressure value based on the plurality of pieces of pressure data and the PPG signal corresponding to each piece of pressure data.

S914: Save and display the blood pressure value.

The blood pressure value includes an SBP and a DBP. The wearable device may save the blood pressure value, and may also save a test time of the blood pressure value.

Optionally, the wearable device may also display the blood pressure value on an interface. The wearable device may wait for an operation performed by the user after displaying the blood pressure value, or may display an option of re-measurement to re-measure the blood pressure, and may also display an option of quitting to end blood pressure measurement.

S915: End the blood pressure measurement and prompt the user that stability of the wearable device is poor in the process if a stability situation does not satisfy a data collection requirement.

If the stability situation of the wearable device does not satisfy the data collection requirement during the pressure application by the user, the blood pressure measurement may be ended, and the user may be prompted with texts or sound that the stability of the wearable device is poor.

S916: Display the option of re-measurement.

The wearable device may also display the option of re-measurement through the human-machine interaction interface, so as to facilitate re-measurement by the user.

S917: Detect a triggering operation performed by the user on the option of re-measurement.

When the wearable device detects the triggering operation performed by the user on the option of re-measurement, S906-S917 described above may be performed.

According to the blood pressure measurement method of a wearable device provided in embodiments of this application, it is determined whether the measurement condition is satisfied before the blood pressure measurement is performed. Measurement is performed based on the measurement condition that is satisfied, which is advantageous in improving accuracy of the blood pressure value. The user is guided to apply the pressure after the blood pressure measurement is enabled, which is advantageous in improving user experience. In addition, the blood pressure value is calculated based on the pressure value and the PPG signal, which is advantageous in ensuring accuracy of the blood pressure value compared with a calculation method using only the PPG signal. During the pressure application by the user, the stability situation of the wearable device may also need to be determined, which is advantageous in ensuring accuracy of data collection, thereby improving the accuracy of the blood pressure value.

In an optional embodiment, the measurement condition may include that the wearable device is in a relatively static state and the wearable device is in good contact with the skin.

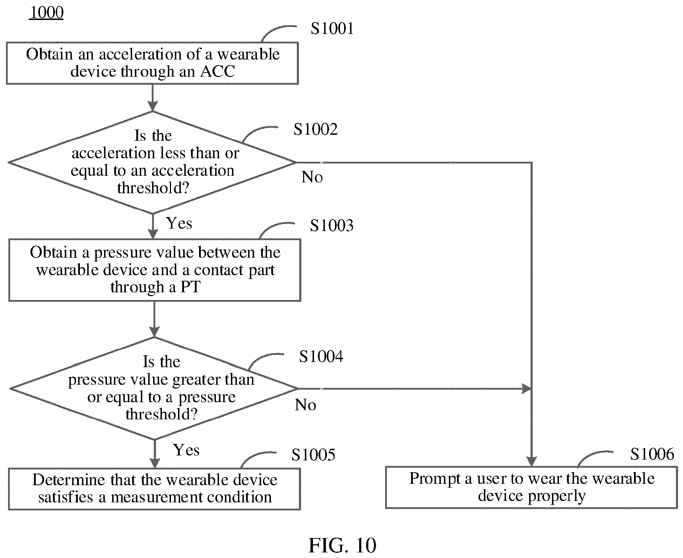

For example, FIG. 10 is a schematic flowchart showing a method 1000 for determining a measurement condition. As shown in FIG. 10, the method 1000 may include the following steps.

S1001: Obtain an acceleration of a wearable device through an ACC.

The wearable device may obtain a signal of the ACC, and data carried by the signal is the acceleration of the wearable device.

S1002: Determine whether the acceleration is less than or equal to an acceleration threshold.

The acceleration threshold is preset, and a specific value of the acceleration threshold is not limited in embodiments of this application.

If the acceleration is less than or equal to the acceleration threshold, it may indicate that the wearable device is in a relatively static state, and another condition may continue to be determined, that is, 1003 is performed. If the acceleration is greater than the acceleration threshold, it may indicate that the wearable device is in a non-relatively static state or in a shaking state. A user may be prompted to wear the wearable device properly, that is, 1006 is performed.

S1003: Obtain a pressure value between the wearable device and a contact part through a PT if the acceleration is less than or equal to the acceleration threshold, where the contact part is a part where the wearable device contacts the user when the wearable device is worn.

The wearable device may obtain a signal of the PT, and data carried by the signal is a pressure value.

The pressure value between the wearable device and the contact part may be configured for determining a contact situation between the wearable device and the contact part.

S1004: Determine whether the pressure value is greater than or equal to a pressure threshold.

The pressure threshold is preset, and a specific value of the pressure threshold is not limited in embodiments of this application.

If the pressure value is greater than or equal to the pressure threshold, it may indicate that the wearable device is in good contact with the contact part (skin or a wrist), that is, a wearing tightness is appropriate, and another condition may continue to be determined, that is, 1005 is performed. If the pressure value is less than the pressure threshold, it may indicate that the wearable device is not in good contact with the contact part (the skin or the wrist), that is, the wearable device is worn loosely, and the user may be prompted to wear the wearable device properly, that is, 1006 is performed.

S1005: It may be determined that the wearable device satisfies the measurement condition if the pressure value is greater than or equal to the pressure threshold.

The wearable device may determine that the wearable device satisfies the measurement condition when the wearable device satisfies the following conditions:

the acceleration is less than or equal to the acceleration threshold, and the pressure value is greater than or equal to the pressure threshold.

S1006: Prompt the user to wear the wearable device properly.

The wearable device may determine that the wearable device does not satisfy the measurement condition and the user is prompted to wear the wearable device properly when the wearable device satisfies at least one of the following conditions:

the acceleration is greater than the acceleration threshold; and/or the pressure value is less than the pressure threshold.

It is to be noted that an execution order of S1002 and S 1004 described above is not limited in embodiments of this application.

If the wearable device satisfies the measurement condition, blood pressure measurement may be performed, that is, S904 is performed. If the wearable device does not satisfy the measurement condition, the user may be continuously prompted to wear the wearable device properly, that is, S902 is performed.

According to the method provided in embodiments of this application, it is determined whether the measurement condition is satisfied before the blood pressure measurement is performed. Measurement is performed based on the measurement condition that is satisfied, which is advantageous in improving accuracy of the blood pressure value.

In an optional embodiment, embodiments of this application further provide a method for adjusting a light emitting region between S908 and S909 described above. At least one of an area size, a position, or a shape of the light emitting region is adjusted to ensure signal quality of a PPG signal.

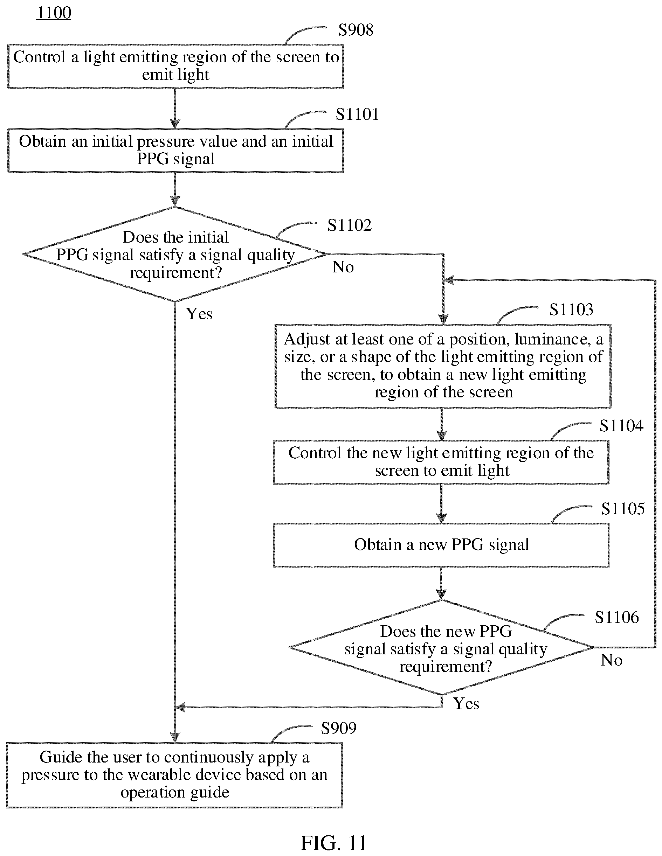

For example, FIG. 11 is a schematic flowchart showing a method 1100 for adjusting a light emitting region. As shown in FIG. 11, the method 1100 may include the following steps.

A wearable device may perform S1101 after performing S908 of controlling a light emitting region of a screen to emit light.

S1101: The wearable device obtains an initial pressure value and an initial PPG signal.

The wearable device may obtain the initial pressure value of a finger touching the screen through a PT, and convert a received optical signal into a PPG signal by using a PD, to obtain the initial PPG signal.

It is to be noted that the initial pressure value and the initial PPG signal are both obtained before a user touches the screen but does not start to apply a pressure. It is to be understood that the initial pressure value and the initial PPG signal are merely examples of a name. This is not limited in embodiments of this application. The initial PPG signal may be understood as a first PPG signal. This is not limited in embodiments of this application.

S1102: Determine whether the initial PPG signal satisfies a signal quality requirement.

The wearable device determines whether the initial PPG signal satisfies the signal quality requirement, that is, determines whether the initial PPG signal under the initial pressure value satisfies the signal quality requirement.

The wearable device may determine in a plurality of manners whether the initial PPG signal satisfies the signal quality requirement.

In a possible implementation, it is determined, by using a waveform feature of the initial PPG signal, whether the signal quality requirement is satisfied.

For example, the wearable device may extract the waveform feature of the initial PPG signal through a feature extraction model, and compare the waveform feature of the initial PPG signal with a preset waveform feature, to determine whether the initial PPG signal satisfies the signal quality requirement.

Optionally, the wearable device may perform denoising and/or filtering on the initial PPG signal before extracting the waveform feature of the initial PPG signal, to ensure accuracy of the extracted waveform feature.

In another possible implementation, it is determined, by using waveform features of different optical signals, whether the initial PPG signal satisfies the signal quality requirement.

For example, the wearable device may use waveforms of a green light signal, a red light signal, and an infrared light signal to perform similarity or correlation comparison to determine whether the initial PPG signal satisfies the signal quality requirement.

If the initial PPG signal satisfies the signal quality requirement, it may indicate that the PPG signal has good quality, and the user may be guided to continuously apply the pressure to the wearable device based on an operation guide, that is, S909 may be performed. If the initial PPG signal does not satisfy the signal quality requirement, it may indicate that the PPG signal has poor quality, and adjustment for the light emitting region of the screen may be prompted, that is, S1103 is performed.

S1103: Adjust at least one of a position, luminance, an area size, or a shape of the light emitting region of the screen, to obtain a new light emitting region of the screen.

The wearable device may determine a manner of the adjustment based on a reason why the PPG signal cannot satisfy the signal quality requirement.

In a possible implementation, if the initial PPG signal does not satisfy the signal quality requirement due to a low perfusion rate of the PPG signal, the wearable device may increase a horizontal distance between the light emitting region of the screen and the PD, that is, change the position of the light emitting region of the screen to obtain the PPG signal that satisfies the signal quality requirement.

In another possible implementation, if the initial PPG signal does not satisfy the signal quality requirement due to an excessively weak PPG signal, the wearable device may increase the luminance of the light emitting region of the screen to obtain the PPG signal that satisfies the signal quality requirement. If the PPG signal is excessively weak, and the luminance of the light emitting region of the screen has reached an adjustable maximum value, the wearable device may reduce the horizontal distance between the light emitting region of the screen and the PD, that is, change the position of the light emitting region of the screen, and improve an intensity of the PPG signal by reducing an optical path, to obtain the PPG signal that satisfies the signal quality requirement.

In still another possible implementation, the wearable device may use a manner of signal inspection. After the finger touches the screen, an exact position and a touch area size of the finger touching the screen are located through a touch sensor, and the horizontal distance between the light emitting region of the screen and the PD is successively changed in the regions touched by the finger. In other words, the position of the light emitting region of the screen is successively changed, the PPG signal of each position is collected, a PPG signal that satisfies the signal quality requirement is selected from the PPG signals, and a position corresponding to the PPG signal is determined as the position of the light emitting region of the screen.

The shape of the light emitting region of the screen may be a circle, an ellipse, a strip, a square, a triangle, an arc, or the like. This is not limited in embodiments of this application. A correspondence may exist between the light emitting region of the screen and the position of the light emitting region of the screen. The correspondence may be a one-to-one relationship, a one-to-many relationship, or a many-to-one relationship. This is not limited in embodiments of this application. The correspondence may be preset. When the wearable device determines the position of the light emitting region of the screen, the shape of the light emitting region of the screen is determined based on the position and the correspondence of the light emitting region of the screen. The quality of the PPG signal varies with the shape of the light emitting region of the screen.

In another possible implementation, if no correspondence exists between the light emitting region of the screen and the position of the light emitting region of the screen, and the PPG signal does not satisfy the signal quality requirement, the wearable device may successively change the shape of the light emitting region of the screen, collect the PPG signal under each shape, select the PPG signal that satisfies the signal quality requirement from the PPG signals, and determine the shape corresponding to the PPG signal as the shape of the light emitting region of the screen.

The area size of the light emitting region of the screen may vary, and the area size of the light emitting region of the screen may be related to the position of the light emitting region of the screen.

In still another possible implementation, if the PPG signal does not satisfy the signal quality requirement, the wearable device may successively change the area size of the light emitting region of the screen based on a zoom-out criterion or a zoom-in criterion, collect the PPG signal under each area size, select the PPG signal that satisfies the signal quality requirement from the PPG signals, and determine the area size corresponding to the PPG signal as the area size of the light emitting region of the screen.

S1104: Control a new light emitting region of the screen to emit light.

The wearable device may use the new light emitting region of the screen as a measurement light source and control the light emitting region to emit an optical signal.

S1105: Obtain a new PPG signal.

The wearable device receives, by using the PD, the optical signal reflected by the finger, and converts the optical signal into the PPG signal to obtain the new PPG signal.

S1106: Determine whether the new PPG signal satisfies the signal quality requirement.

If the new PPG signal satisfies the signal quality requirement, the user may be guided to continuously apply the pressure to the wearable device based on the operation guide, that is, S909 may be performed. If the new PPG signal still does not satisfy the signal quality requirement, at least one of the position, the luminance, the area size, or the shape of the light emitting region of the screen may continue to be adjusted, that is, S1103 may be performed.

According to the method for adjusting a light emitting region provided in this embodiment of this application, at least one of the position, the luminance, the area size, or the shape of the light emitting region of the screen may be adjusted to ensure that the PPG signal that satisfies the signal quality requirement may be collected, which is advantageous in improving accuracy of subsequent blood pressure value measurement.

In an optional embodiment, in S913 described above, the blood pressure value is obtained based on the pressure data and the PPG signal corresponding to the pressure data. If a plurality of pieces of pressure data exist, a plurality of PPG signals corresponding to the pressure data exist. The wearable device may obtain the blood pressure value based on the plurality of pressure values and the plurality of PPG signals.

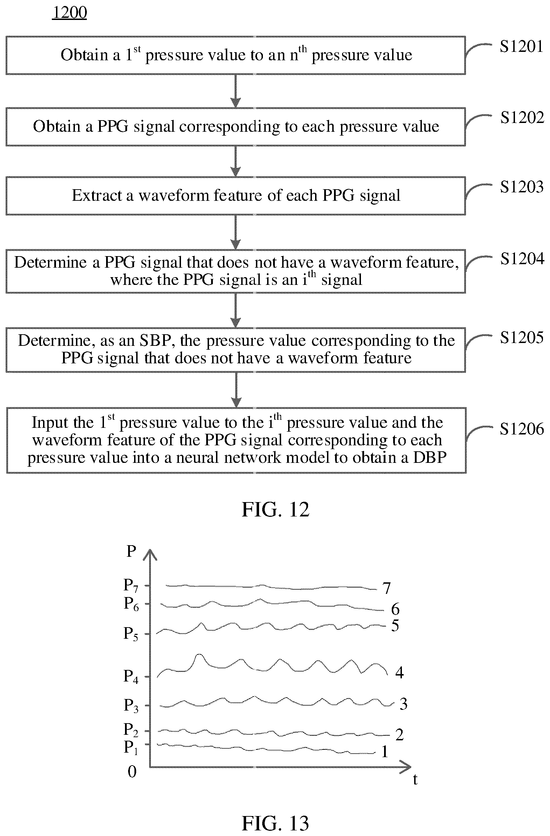

For example, FIG. 12 is a schematic flowchart showing a method 1200 for calculating a blood pressure value. As shown in FIG. 12, the method 1200 may include the following steps.

S1201: Obtain a 1^{st} pressure value to an n^{th} pressure value during continuous pressing by a user.

During the pressure application by the user, a pressure increases as a pressure application process progresses. The 1^{st} pressure value is the smallest one of the n pressure values, and the n^{th} pressure value is the largest one of the n pressure values. A value of n may be 3, or may be 5, and may also be 10. This is not limited in embodiments of this application.

S1202: Obtain a PPG signal corresponding to each pressure value.

A wearable device may also obtain the PPG signal under the pressure value while obtaining the 1^{st} pressure value. In other words, the wearable device controls a light emitting region of a screen to emit light, receives, through a PD, an optical signal reflected by a fingertip, converts the reflected optical signal into an electrical signal, and then converts the electrical signal into the PPG signal for blood pressure measurement. By analogy, the wearable device may obtain the PPG signal corresponding to each pressure value, and then obtain n PPG signals.

Different pressure values correspond to different PPG signals, and waveforms of the PPG signals under different pressure values are different. When the pressure value of the finger pressing the wearable device is relatively small, skin of the finger is not in good contact with a plane, a waveform signal detected by the PPG sensor is relatively weak, and a pulse pulsation amplitude of the waveform of the PPG signal is relatively small. As the pressure gradually increases, contact between the finger and the screen is increasingly good, and the optical signal emitted by the light emitting region of the screen may be more effectively incident on the finger. The PD may receive increasingly more optical signals reflected by the finger, and the pulse pulsation amplitude of the waveform of the PPG signal becomes increasingly larger and reaches a maximum value. Afterward, as the pressure gradually increases, the pressure affects pulsation of blood vessels in the finger, causing a pulsation magnitude to be constantly limited and blood vessels to be squeezed. The pulse pulsation amplitude of the waveform of the PPG signal gradually decreases until a waveform curve becomes straight and has no pulse shape. In this embodiment of this application, the pressure applied by the user may cause the pulse pulsation amplitude of the waveform of the PPG signal to change from a small value to a large value until a maximum value, and then from the large value to the small value until the waveform curve becomes straight.

For example, FIG. 13 is a schematic diagram showing waveform curve changes of a PPG signal. As shown in FIG. 13, n is equal to 7. When n = 1, a pressure value is P₁. When n = 2, the pressure value is P₂. When n = 3, the pressure value is P₃. When n = 4, the pressure value is P₄. When n = 5, the pressure value is P₅. When n = 6, the pressure value is P₆. When n = 7, the pressure value is P₇. P₁ < P₂ < P₃ < P₄ < P₅ < P₆ < P₇. At a pressure value (P₁, P₂, P₃, P₄, P₅, P₆, or P₇), the waveform of the PPG signal constantly changes with time. As the pressure value continuously increases, that is, from P₁ to P₇, the pulse pulsation amplitude of the waveform of the PPG signal changes from a small value to a large value until a maximum value, and then from the large value to the small value until the waveform curve becomes straight. It may be seen from FIG. 13 that the pulse pulsation amplitude of the waveform of the PPG signal corresponding to a 4^{th} pressure value is the largest. The waveform curve of the PPG signal corresponding to a 7^{th} pressure value is straight and has no pulse shape.

S1203: Extract a waveform feature of each PPG signal.

The wearable device may extract the waveform feature of each PPG signal through a feature extraction model. The waveform form of each PPG signal includes a physiological feature of a human body. The waveform feature of each PPG signal may include at least one of a main peak height of a PPG waveform, a height of a dicrotic notch, an amplitude height of a replay wave, a pulse conduction time, diastole, systole, and rise time and fall time of the waveform.

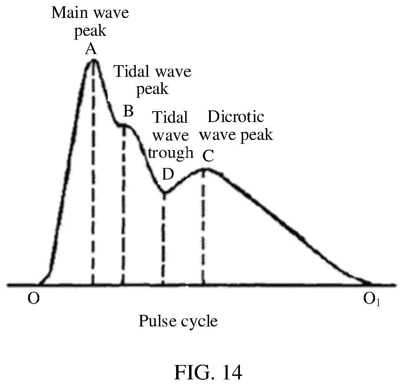

For example, FIG. 14 is a schematic diagram showing a waveform of a PPG signal. As shown in FIG. 14, the waveform of the PPG signal is described by using one pulse cycle as an example. A point O is a starting point of a cardiac ejection phase. A point A is a highest point of an aortic pressure and reflects the maximum pressure and volume in the artery. A point B is a stopping point of left ventricular ejection, is a peak point of a reflection wave and also referred to as a tidal wave peak, and reflects a magnitude of tension, compliance, and peripheral resistance of the artery. A point D is a tidal wave trough point, that is, a boundary point between cardiac contraction and relaxation, and mainly reflects the magnitude of the peripheral resistance.

An OA segment waveform is an ascending branch of the pulse waveform, which is caused by expansion of a vascular wall as a result of left ventricular ejection and a rapid rise of arterial blood pressure during sudden expansion of an arterial wall. A cardiac output, an ejection velocity, and a resistance are main factors affecting the amplitude and a slope of the ascending branch, which are generally expressed by a slope and an amplitude of rising of a pulse wave waveform. A larger cardiac output, a faster ejection velocity, higher aortic elasticity, and smaller resistance lead to a larger slope and a higher amplitude. On the contrary, the slope is relatively small, and the amplitude is relatively low. An AD segment waveform is an anterior segment of a descending branch, which is caused by a process in which the ejection velocity begins to decrease in a later stage of the ventricular ejection, causing blood flow around the aorta to be greater than blood flow into the aorta, the aorta changes from expansion to retraction, and the arterial blood pressure gradually decreases. A DO₁ segment waveform is a posterior segment of the descending branch, also referred to as a dicrotic wave, which is formed by ventricular dilation, a continuous decrease in the arterial blood pressure, and reflux of blood in the aorta to a ventricle. This reflects a functional status of the aorta, vascular elasticity, and a blood flow state.

The wearable device may extract waveform features such as the main peak height of the waveform of the PPG signal, the height of the dicrotic notch, the amplitude height of the replay wave, the pulse conduction time, the diastole, the systole, and the rise time and fall time of the waveform.

S1204: Determine, from n PPG signals, a PPG signal that does not have a waveform feature, where the PPG signal is an i^{th} signal.

The wearable device may determine, from the n PPG signals based on the waveform feature of each PPG signal, the PPG signal that does not have a waveform feature. The PPG signal that does not have a waveform feature is an i^{th} PPG signal, where i is greater than or equal to 1, and i is less than or equal to n. The n PPG signals may be referred to as a plurality of second PPG signals, and the PPG signal that does not have a waveform feature may be referred to as a target second PPG signal. This is not limited in embodiments of this application.

For example, in the foregoing example shown in FIG. 13, a 7^{th} PPG signal is the PPG signal that does not have a waveform feature, where i = 7.

S1205: Determine, as a systolic blood pressure (systolic blood pressure, SBP), a pressure value corresponding to the PPG signal that does not have a waveform feature.

The pressure value corresponding to the PPG signal that does not have a waveform feature is an i^{th} pressure value, and the wearable device may determine the i^{th} pressure value as the systolic blood pressure.

For example, in the foregoing example shown in FIG. 13, the 7^{th} PPG signal is the PPG signal that does not have a waveform feature, where i = 7. The wearable device may determine the 7^{th} pressure value as the systolic blood pressure.

S 1206: Input the 1^{st} pressure value to the i^{th} pressure value and the waveform feature of the PPG signal corresponding to each pressure value into a neural network model 1, to obtain a diastolic blood pressure (diastolic blood pressure, DBP).

The wearable device may input the 1^{st} pressure value and the waveform feature of the PPG signal corresponding to the 1^{st} pressure value to the i^{th} pressure value and the waveform feature of the PPG signal corresponding to the i^{th} pressure value into the neural network model 1, to obtain the DBP.

It is to be noted that input to the neural network model 1 is the pressure value and the waveform feature of the PPG signal corresponding to the pressure value, and output of the neural network model is the DBP. The neural network model 1 may be trained by using a large quantity of historical data.

According to the method for calculating a blood pressure value provided in embodiments of this application, the SBP (that is, a high pressure) of the blood pressure may be directly measured through the PT, and the DBP (that is, a low pressure) is calculated through the waveform feature presented by the PPG signal. Compared with the blood pressure value calculated through regression or another calculation method, accuracy of the blood pressure value is improved.

In an optional embodiment, in S914 described above, the wearable device may display the blood pressure value.

The wearable device may display the measured blood pressure value on an interface, may also display a pressure curve on the interface to guide the user to continuously apply the pressure based on the pressure curve, and may also display the waveform of the PPG signal.

For example, FIG. 15 is a schematic diagram showing a blood pressure measurement interface. As shown in an interface a in FIG. 15, a wearable device may use texts to guide a user to touch a fingerprint region with a finger. The text content may be "place the finger at an icon position, and please press to measure". The wearable device may guide, through a pressure curve, the user to continuously apply a pressure perpendicular to the wearable device to the wearable device, and display upper and lower limits of the pressure curve on an interface to regulate a behavior of applying the pressure by the user.

As shown in an interface b in FIG. 15, when the wearable device detects that the user touches the fingerprint region, the user may be guided to continuously apply the pressure to the wearable device through sound "please apply a downward pressure to a watch body to keep a black spot on a pressure curve during measurement", so that a pressure transducer at a bottom of the wearable device can detect a continuous pressing pressure change. During the pressure application by the user, a countdown time for the pressure application may also be displayed on the interface, for example, a countdown of 10 seconds (seconds), to prompt the user of a duration for the continuous pressure application. In addition, a prompting mode such as a color or sound may also be displayed on the interface, to guide the user to correctly apply the pressure and ensure a measurement requirement. In other words, during the measurement, the application of the downward pressure is to be maintained to avoid a measurement error caused by skewness or warpage of the wearable device. During the pressure application by the user, the wearable device collects pressure data, and obtains a PPG signal of a fingertip under the pressure data.

As shown in an interface c in FIG. 15, after the wearable device determines an SBP and a DBP according to the foregoing method 1200, a blood pressure value is displayed on the interface, that is, systolic blood pressure: 120 millimetres of mercury (millimetre of mercury, mmHg), and diastolic blood pressure: 70 mmHg.

The sequence number of the foregoing processes does not mean the order of execution, and the order of execution of each process is to be determined by the function and internal logic thereof, and is not to constitute any limitation on the implementation process of embodiments of this application.

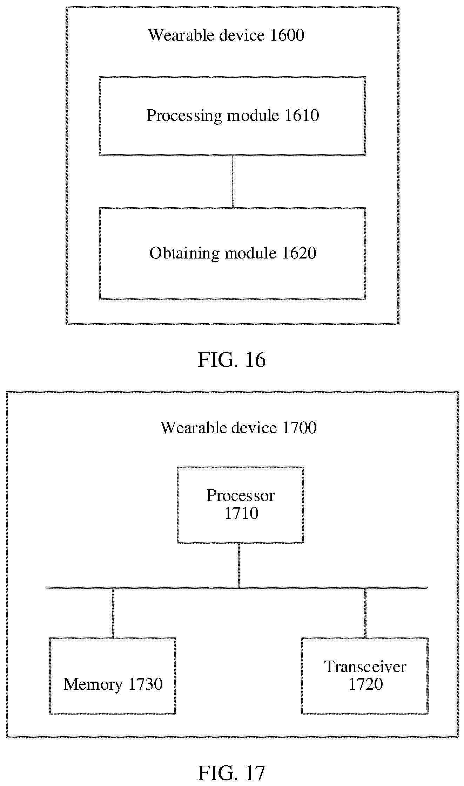

The method provided in embodiments of this application is described in detail with reference to FIG. 1 to FIG. 15 above, and a device provided in embodiments of this application is to be described in detail with reference to FIG. 16 and FIG. 17.

FIG. 16 is a schematic flowchart showing a wearable device 1600 according to an embodiment of this application. The wearable device 1600 includes a processing module 1610 and an obtaining module 1620. The wearable device 1600 may be configured to perform each of the foregoing methods. For example, the wearable device 1600 may be configured to perform at least one of the method 900, the method 1000, the method 1100, and the method 1200 described above.

It is to be understood that the wearable device 1600 herein is embodied in a form of a functional module. The term "module" herein may be an application specific integrated circuit (application specific integrated circuit, ASIC), an electronic circuit, a processor (for example, a shared processor, a special purpose processor, or a group processor) configured to execute one or more software or firmware programs, a memory, a combinational logic circuit, and/or another suitable component that supports the described function. In an optional example, a person skilled in the art may understand that the wearable device 1600 may be specifically the wearable device in the foregoing method embodiments, or that the functions of the wearable device in the foregoing method embodiments may be integrated in the wearable device 1600. The wearable device 1600 may be configured to perform each process and/or step corresponding to the wearable device in the foregoing method embodiments. To avoid repetitions, details are not described herein again.

The foregoing wearable device 1600 has a function of implementing corresponding steps performed by the wearable device in the foregoing method embodiments. The foregoing function may be implemented by hardware, or may be implemented by hardware executing corresponding software. The hardware or software includes one or more modules corresponding to the foregoing functions.

In this embodiment of this application, the wearable device 1600 in FIG. 16 may alternatively be a chip or a chip system, for example, a system on chip (system on chip, SoC).

FIG. 17 is a schematic block diagram of another wearable device 1700 according to an embodiment of this application. The wearable device 1700 includes a processor 1710, a transceiver 1720, and a memory 1730. The processor 1710, the transceiver 1720, and the memory 1730 communicate with each other through an internal connection path, the memory 1730 is configured to store instructions, and the processor 1720 is configured to execute the instructions stored in the memory 1730, to control the transceiver 1720 to send and/or receive signals.

It is to be understood that the wearable device 1700 may be specifically the wearable device in the foregoing method embodiments, or the functions of the wearable device in the foregoing method embodiments may be integrated in the wearable device 1700. The wearable device 1700 may be configured to perform each step and/or process corresponding to the wearable device in the foregoing method embodiments. Optionally, the memory 1730 may include a read-only memory and a random access memory, and provide an instruction and data to the processor. A part of the memory may further include a non-volatile random access memory. For example, the memory may further store information about a device type. The processor 1710 may be configured to execute the instruction stored in the memory, and when the processor executes the instruction, the processor may perform each step and/or process corresponding to the wearable device in the foregoing method embodiment.

It is to be understood that, in embodiments of this application, the processor 1710 may be a central processing unit (central processing unit, CPU). The processor may also be another general-purpose processor, a digital signal processor (DSP), an application-specific integrated circuit (ASIC), a field-programmable gate array (FPGA) or another programmable logic device, a discrete gate or transistor logic device, a discrete hardware component, or the like. The general-purpose processor may be a microprocessor, the processor may also be any conventional processor, or the like.

During implementation, the steps of the foregoing method may be completed through an integrated logic circuit of hardware or an instruction in the form of software in the processor. The steps of the method disclosed in combination with embodiments of this application may be directly performed by a hardware processor, or may be performed through a combination of hardware and software modules in the processor. A software module may be located in a mature storage medium in the art, such as a random access memory, a flash memory, a read-only memory, a programmable read-only memory, an electrically erasable programmable memory, or a register. The storage medium is located in the memory. The processor executes the instruction in the memory and performs the steps of the foregoing method in combination with hardware thereof. To avoid repetition, details are not described herein again.

This application further provides a computer-readable storage medium. The computer-readable storage medium is configured to store a computer program. The computer program is configured for implementing the method corresponding to the wearable device in the foregoing method embodiment.

This application also provides a chip system. The chip system is configured to support the wearable device in the foregoing method embodiment to implement the functions shown in embodiments of this application.

This application further provides a computer program product. The computer program product includes computer program code (which may also be referred to as code or an instruction). When the computer program code runs on a computer, the computer may perform the method corresponding to the wearable device shown in the foregoing method embodiment.

A person of ordinary skill in the art may be aware that, in combination with the examples described in embodiments disclosed in this specification, modules and algorithm steps can be implemented by electronic hardware or a combination of computer software and electronic hardware. Whether the functions are executed by hardware or software depends on specific applications and design constraint conditions of the technical solutions. A person skilled in the art may use different methods to implement the described functions for each particular application, but it is not to be considered that such implementation goes beyond the scope of this application.

It may be clearly understood by a person skilled in the art that, for the purpose of convenient and brief description, for a detailed operating process of the system, apparatus, and module described above, reference may be made to the corresponding process in the foregoing method embodiment. Details are not described herein again.

In the several embodiments provided in this application, it is to be understood that the disclosed system, apparatus, and method may be implemented in other manners. For example, the apparatus embodiments described above are merely examples. For example, division of modules is merely logical function division and may be another division manner during actual implementation. For example, a plurality of modules or components may be combined or integrated into another system, or some features may be omitted or not executed. In addition, the displayed or discussed mutual coupling or direct coupling or communication connection may be implemented by using some interfaces. The indirect coupling or communication connection between the apparatuses or modules may be electrical, mechanical, or in other forms.

The modules described as separate components may or may not be physically separated, and the components displayed as modules may or may not be physical modules, that is, may be located in one place or may be distributed on a plurality of network modules. Some or all of the modules may be selected according to actual needs to achieve the objectives of the solutions of embodiments.

In addition, functional modules in embodiments of this application may be integrated into one processing module, or the functional modules may exist alone physically, or two or more modules may be integrated into one module.

When the functions are implemented in the form of a software functional module and sold or used as an independent product, the functions may be stored in a computer-readable storage medium. Based on such an understanding, the technical solutions of this application essentially, or the part contributing to the prior art, or a part of the technical solutions may be implemented in the form of a software product. The computer software product is stored in a storage medium, and includes several instructions for enabling a computer device (which may be a personal computer, a server, a network device, or the like) to perform all or part of the steps of the method described in embodiments of this application. The foregoing storage medium includes: any medium that may store program code, such as a USB flash drive, a removable hard disk, a read-only memory (read-only memory, ROM), a random access memory (random access memory, RAM), a magnetic disk, or a compact disc.

The invention is defined by the appended claims.

## Claims

1. A blood pressure measurement method of a wearable device (100, 1700), wherein the wearable device (100, 1700) comprises a pressure transducer (120), PT, a display screen (161), and a photo diode, PD, (131); and
the method comprises:
emitting an optical signal through a first region of the display screen (161);
converting, into a first PPG signal, the optical signal received by the PD (131), wherein the optical signal emitted by the first region is absorbed and scattered by skin tissue to form a reflected signal, and the PD converts the reflected signal into the first PPG signal;
determining whether the first PPG signal satisfies a signal quality requirement by calculating a perfusion rate of the first PPG signal and comparing the perfusion rate to a preset perfusion rate or by calculating an intensity of the first PPG signal and comparing the intensity to a preset intensity;
if the first PPG signal satisfies the signal quality requirement:
obtaining a pressure value collected by the PT (120); and
determining a blood pressure value of a wearer based on the pressure value and the first PPG signal;
if the first PPG signal does not satisfy a signal quality requirement:
emitting the optical signal through a second region of the display screen (161), wherein the second region and the first region are different in at least one of the following features: a position, a luminance, an area size, or a shape on the display screen (161);
converting, into a second PPG signal, the optical signal received by the PD (131);
determining whether the second PPG signal satisfies the signal quality requirement by calculating a perfusion rate of the second PPG signal and comparing the perfusion rate to the preset perfusion rate or by calculating an intensity of the second PPG signal and comparing the intensity to a preset intensity;
if the second PPG signal satisfies the signal quality requirement:
obtaining a pressure value collected by the PT (120) when the second PPG signal satisfies the signal quality requirement; and
determining a blood pressure value of a wearer based on the pressure value and the second PPG signal.

2. The method according to claim 1, wherein, if the first PPG signal does not satisfy a signal quality requirement comprises: a perfusion rate of the first PPG signal is less than or equal to a preset perfusion rate,
a distance between a projection region of the PD (131) on the display screen (161) and the second region is greater than a distance between the projection region of the PD (131) on the display screen (161) and the first region.

3. The method according to claim 1, wherein, if the first PPG signal does not satisfy a signal quality requirement comprises: an intensity of the first PPG signal is less than or equal to a preset intensity,
the luminance of the second region is greater than the luminance of the first region.

4. The method according to claim 3, wherein the luminance of the second region is a maximum value; and
the method further comprises:
emitting the optical signal through a third region of the display screen (161) if the second PPG signal does not satisfy the signal quality requirement, wherein a distance between a projection region of the PD (131) on the display screen (161) and the third region is less than a distance between the projection region of the PD (131) on the display screen (161) and the first region;
converting, into a third PPG signal, the optical signal received by the PD (131);
obtaining the pressure value collected by the PT (120) when the third PPG signal satisfies the signal quality requirement; and
determining the blood pressure value of the wearer based on the pressure value and the third PPG signal.

5. The method according to claim 1, wherein the shape of the second region is different from the shape of the first region; or
the area size of the second region is different from the area size of the first region.

6. The method according to any one of claims 1 to 5, wherein the pressure value comprises a plurality of pressure values, the second PPG signal comprises a plurality of second PPG signals, the plurality of pressure values are in a one-to-one correspondence with the plurality of second PPG signals, and a collection moment of each of the plurality of pressure values is same as that of a corresponding one of the second PPG signals; and
the determining a blood pressure value of a wearer based on the pressure value and the second PPG signal comprises:
extracting a waveform feature of each of the plurality of second PPG signals; and
determining, if a target second PPG signal whose waveform feature is less than or equal to a preset waveform feature exists in the plurality of second PPG signals, a pressure value corresponding to the target second PPG signal as a systolic blood pressure, SBP, in the blood pressure value of the wearer.

7. The method according to any one of claims 1 to 6, wherein the pressure value comprises a plurality of pressure values, the second PPG signal comprises a plurality of second PPG signals, the plurality of pressure values are in a one-to-one correspondence with the plurality of second PPG signals, and a collection moment of each of the plurality of pressure values is same as that of a corresponding one of the second PPG signals; and
the determining a blood pressure value of a wearer based on the pressure value and the second PPG signal comprises:
extracting a waveform feature of each of the plurality of second PPG signals; and
inputting the plurality of pressure values and the waveform features of the plurality of second PPG signals into a neural network model, wherein the neural network model is trained based on a historical pressure value and a waveform feature of a historical PPG signal, and is configured to measure blood pressure; and
determining output of the neural network model as a diastolic blood pressure DBP in the blood pressure value of the wearer.

8. The method according to any one of claims 1 to 7, wherein before the obtaining a pressure value collected by the PT (120), the method further comprises:
outputting an operation guide, wherein the operation guide is configured to guide the wearer to apply a pressure perpendicular to a contact surface between the wearable device (100, 1700) and human skin to the wearable device (100, 1700) in a preset pressing region, and the operation guide comprises a pressure value required for the wearer to press.

9. The method according to claim 8, further comprising:
ending, during the pressure application by the wearer, blood pressure measurement if the following condition is satisfied:
an acceleration of the wearable device (100, 1700) is greater than an acceleration threshold; and/or
an angular velocity of the wearable device (100, 1700) is greater than an angular velocity threshold.

10. The method according to claim 8 or 9, further comprising:
displaying a first interface, wherein the first interface is configured to display a pressure curve, prompt information, and a waveform of the second PPG signal, the pressure curve is the operation guide, and the prompt information is configured for prompting a pressing duration of the wearer.

11. The method according to any one of claims 1 to 10, wherein before the emitting an optical signal through a first region of the display screen (161), the method further comprises:
determining whether the wearable device (100, 1700) satisfies a blood pressure measurement condition; and
the emitting an optical signal through a first region of the display screen (161) comprises:
emitting the optical signal through the first region of the display screen (161) when the blood pressure measurement condition is satisfied.

12. The method according to claim 11, wherein the blood pressure measurement condition comprises at least one of the following:
the acceleration of the wearable device (100, 1700) is less than or equal to the acceleration threshold, the pressure value is greater than or equal to a pressure threshold, or the angular velocity of the wearable device (100, 1700) is less than or equal to the angular velocity threshold, wherein
the acceleration is configured for representing a contact situation between the wearable device (100, 1700) and the wearer, and the pressure value is configured for representing a tightness for wearing the wearable device (100, 1700).

13. The method according to any one of claims 1 to 12, wherein the display screen (161) is supported by a screen bracket, the screen bracket comprises an optical window, the PD (131) is directly below the optical window, a preset distance is defined between the PD (131) and the optical window, the PT (120) is located at a bottom of the wearable device (100, 1700) and is configured to collect a pressure value between the wearable device (100, 1700) and a wearing part, and the wearing part is a contact part between the wearable device (100, 1700) and the wearer.

14. A wearable device (100, 1700), comprising: a processor (1710), wherein the processor (1710) is coupled to a memory (1730), the memory (1730) is configured to store a computer program, and when the processor (1710) invokes the computer program, the wearable device (100, 1700) is caused to perform the method according to any one of claims 1 to 13.

15. A computer-readable storage medium, configured to store a computer program, wherein the computer program comprises an instruction for implementing the method according to any one of claims 1 to 13.

## Patentansprüche

1. Blutdruckmessverfahren einer tragbaren Vorrichtung (100, 1700), wobei die tragbare Vorrichtung (100, 1700) einen Druckwandler (120), PT, einen Anzeigebildschirm (161) und eine Fotodiode, PD, (131) umfasst; und
das Verfahren umfasst:
Emittieren eines optischen Signals durch einen ersten Bereich des Anzeigebildschirms (161);
Umwandeln des von der PD (131) empfangenen optischen Signals in ein erstes PPG-Signal, wobei das vom ersten Bereich emittierte optische Signal von Hautgewebe absorbiert und gestreut wird, um ein reflektiertes Signal zu bilden, und die PD das reflektierte Signal in das erste PPG-Signal umwandelt;
Bestimmen, ob das erste PPG-Signal eine Signalqualitätsanforderung erfüllt, durch Berechnen einer Perfusionsrate des ersten PPG-Signals und Vergleichen der Perfusionsrate mit einer voreingestellten Perfusionsrate oder durch Berechnen einer Intensität des ersten PPG-Signals und Vergleichen der Intensität mit einer voreingestellten Intensität;
wenn das erste PPG-Signal die Signalqualitätsanforderung erfüllt:
Erhalten eines vom PT (120) erfassten Druckwertes; und
Bestimmen eines Blutdruckwertes eines Trägers basierend auf dem Druckwert und dem ersten PPG-Signal;
wenn das erste PPG-Signal eine Signalqualitätsanforderung nicht erfüllt:
Emittieren des optischen Signals durch einen zweiten Bereich des Anzeigebildschirms (161), wobei sich der zweite Bereich und der erste Bereich in mindestens einem der folgenden Merkmale unterscheiden: einer Position, einer Leuchtdichte, einer Flächengröße oder einer Form auf dem Anzeigebildschirm (161);
Umwandeln des von der PD (131) empfangenen optischen Signals in ein zweites PPG-Signal;
Bestimmen, ob das zweite PPG-Signal die Signalqualitätsanforderung erfüllt, durch Berechnen einer Perfusionsrate des zweiten PPG-Signals und Vergleichen der Perfusionsrate mit der voreingestellten Perfusionsrate oder durch Berechnen einer Intensität des zweiten PPG-Signals und Vergleichen der Intensität mit einer voreingestellten Intensität;
wenn das second PPG-Signal die Signalqualitätsanforderung erfüllt:
Erhalten eines vom PT (120) erfassten Druckwertes, wenn das zweite PPG-Signal die Signalqualitätsanforderung erfüllt; und
Bestimmen eines Blutdruckwertes eines Trägers basierend auf dem Druckwert und dem zweiten PPG-Signal.

2. Verfahren nach Anspruch 1, wobei, wenn das erste PPG-Signal eine Signalqualitätsanforderung nicht erfüllt, umfasst: eine Perfusionsrate des ersten PPG-Signals ist kleiner als oder gleich einer voreingestellten Perfusionsrate,
ein Abstand zwischen einem Projektionsbereich der PD (131) auf dem Bildschirm (161) und dem zweiten Bereich ist größer als ein Abstand zwischen dem Projektionsbereich der PD (131) auf dem Bildschirm (161) und dem ersten Bereich.

3. Verfahren nach Anspruch 1, wobei, falls das erste PPG-Signal eine Signalqualitätsanforderung nicht erfüllt, dies umfasst: eine Intensität des ersten PPG-Signals ist kleiner als oder gleich einer voreingestellten Intensität,
die Leuchtdichte des zweiten Bereichs ist größer als die Leuchtdichte des ersten Bereichs.

4. Verfahren nach Anspruch 3, wobei die Leuchtdichte des zweiten Bereichs ein Maximalwert ist; und
das Verfahren umfasst ferner:
Aussenden des optischen Signals durch einen dritten Bereich des Bildschirms (161), falls das zweite PPG-Signal die Signalqualitätsanforderung nicht erfüllt, wobei ein Abstand zwischen einem Projektionsbereich der PD (131) auf dem Bildschirm (161) und dem dritten Bereich kleiner als ein Abstand zwischen dem Projektionsbereich der PD (131) auf dem Bildschirm (161) und dem ersten Bereich ist;
Umwandeln des von der PD (131) empfangenen optischen Signals in ein drittes PPG-Signal;
Erhalten des durch den PT (120) erfassten Druckwerts, wenn das dritte PPG-Signal die Signalqualitätsanforderung erfüllt; und
Bestimmen des Blutdruckwerts des Trägers basierend auf dem Druckwert und dem dritten PPG-Signal.

5. Verfahren nach Anspruch 1, wobei sich die Form des zweiten Bereichs von der Form des ersten Bereichs unterscheidet; oder
die Flächengröße des zweiten Bereichs unterscheidet sich von der Flächengröße des ersten Bereichs.

6. Verfahren nach einem der Ansprüche 1 bis 5, wobei der Druckwert eine Vielzahl von Druckwerten umfasst, das zweite PPG-Signal eine Vielzahl von zweiten PPG-Signalen umfasst, die Vielzahl von Druckwerten in einer Eins-zu-eins-Entsprechung mit der Vielzahl von zweiten PPG-Signalen steht und ein Erfassungszeitpunkt von jedem der Vielzahl von Druckwerten derselbe ist wie der eines entsprechenden der zweiten PPG-Signale; und
das Bestimmen eines Blutdruckwerts eines Trägers basierend auf dem Druckwert und dem zweiten PPG-Signal umfasst:
Extrahieren eines Wellenformmerkmals von jedem der Vielzahl von zweiten PPG-Signalen; und
Bestimmen, falls ein Ziel-zweites PPG-Signal, dessen Wellenformmerkmal kleiner als oder gleich einem voreingestellten Wellenformmerkmal ist, in der Vielzahl von zweiten PPG-Signalen existiert, eines Druckwerts, der dem Ziel-zweiten PPG-Signal entspricht, als einen systolischen Blutdruck, SBP, in dem Blutdruckwert des Trägers.

7. Verfahren nach einem der Ansprüche 1 bis 6, wobei der Druckwert eine Vielzahl von Druckwerten umfasst, das zweite PPG-Signal eine Vielzahl von zweiten PPG-Signalen umfasst, die Vielzahl von Druckwerten in einer Eins-zu-eins-Entsprechung mit der Vielzahl von zweiten PPG-Signalen steht und ein Erfassungszeitpunkt jedes der Vielzahl von Druckwerten derselbe ist wie der eines entsprechenden der zweiten PPG-Signale; und
das Bestimmen eines Blutdruckwerts eines Trägers basierend auf dem Druckwert und dem zweiten PPG-Signal umfasst:
Extrahieren eines Wellenformmerkmals jedes der Vielzahl von zweiten PPG-Signalen; und
Eingeben der Vielzahl von Druckwerten und der Wellenformmerkmale der Vielzahl von zweiten PPG-Signalen in ein neuronales Netzwerkmodell, wobei das neuronale Netzwerkmodell basierend auf einem historischen Druckwert und einem Wellenformmerkmal eines historischen PPG-Signals trainiert wird und konfiguriert ist, um den Blutdruck zu messen; und
Bestimmen der Ausgabe des neuronalen Netzwerkmodells als einen diastolischen Blutdruck DBP im Blutdruckwert des Trägers.

8. Verfahren nach einem der Ansprüche 1 bis 7, wobei das Verfahren vor dem Erhalten eines durch den PT (120) erfassten Druckwerts ferner umfasst:
Ausgeben einer Bedienungshilfe, wobei die Bedienungshilfe konfiguriert ist, um den Träger anzuleiten, einen Druck senkrecht zu einer Kontaktfläche zwischen der tragbaren Vorrichtung (100, 1700) und menschlicher Haut auf die tragbare Vorrichtung (100, 1700) in einem voreingestellten Druckbereich auszuüben, und die Bedienungshilfe einen für das Drücken durch den Träger erforderlichen Druckwert umfasst.

9. Verfahren nach Anspruch 8, ferner umfassend:
Beenden der Blutdruckmessung während der Druckausübung durch den Träger, wenn die folgende Bedingung erfüllt ist:
eine Beschleunigung der tragbaren Vorrichtung (100, 1700) größer als ein Beschleunigungsschwellenwert ist; und/oder
eine Winkelgeschwindigkeit der tragbaren Vorrichtung (100, 1700) größer als ein Winkelgeschwindigkeitsschwellenwert ist.

10. Verfahren nach Anspruch 8 oder 9, ferner umfassend:
Anzeigen einer ersten Benutzeroberfläche, wobei die erste Benutzeroberfläche konfiguriert ist, um eine Druckkurve, Hinweisinformationen und eine Wellenform des zweiten PPG-Signals anzuzeigen, wobei die Druckkurve die Bedienungshilfe ist und die Hinweisinformationen zum Hinweisen auf eine Druckdauer des Trägers konfiguriert sind.

11. Verfahren nach einem der Ansprüche 1 bis 10, wobei das Verfahren vor dem Emittieren eines optischen Signals durch einen ersten Bereich des Bildschirms (161) ferner umfasst:
Bestimmen, ob die tragbare Vorrichtung (100, 1700) eine Blutdruckmessbedingung erfüllt; und
das Aussenden eines optischen Signals durch einen ersten Bereich des Anzeigebildschirms (161) umfasst:
Aussenden des optischen Signals durch den ersten Bereich des Anzeigebildschirms (161), wenn die Blutdruckmessbedingung erfüllt ist.

12. Verfahren nach Anspruch 11, wobei die Blutdruckmessbedingung mindestens eines der Folgenden umfasst:
die Beschleunigung der tragbaren Vorrichtung (100, 1700) kleiner oder gleich dem Beschleunigungsschwellenwert ist, der Druckwert größer oder gleich einem Druckschwellenwert ist oder die Winkelgeschwindigkeit der tragbaren Vorrichtung (100, 1700) kleiner oder gleich dem Winkelgeschwindigkeitsschwellenwert ist, wobei
die Beschleunigung dazu konfiguriert ist, eine Kontaktsituation zwischen der tragbaren Vorrichtung (100, 1700) und dem Träger darzustellen, und der Druckwert dazu konfiguriert ist, eine Straffheit beim Tragen der tragbaren Vorrichtung (100, 1700) darzustellen.

13. Verfahren nach einem der Ansprüche 1 bis 12, wobei der Anzeigebildschirm (161) durch eine Bildschirmhalterung gestützt wird, wobei die Bildschirmhalterung ein optisches Fenster umfasst, die PD (131) sich direkt unter dem optischen Fenster befindet, ein voreingestellter Abstand zwischen der PD (131) und dem optischen Fenster definiert ist, der PT (120) sich an einer Unterseite der tragbaren Vorrichtung (100, 1700) befindet und dazu konfiguriert ist, einen Druckwert zwischen der tragbaren Vorrichtung (100, 1700) und einem tragenden Teil zu erfassen, und der tragende Teil ein Kontaktteil zwischen der tragbaren Vorrichtung (100, 1700) und dem Träger ist.

14. Tragbare Vorrichtung (100, 1700), umfassend: einen Prozessor (1710), wobei der Prozessor (1710) mit einem Speicher (1730) gekoppelt ist, wobei der Speicher (1730) dazu konfiguriert ist, ein Computerprogramm zu speichern, und wenn der Prozessor (1710) das Computerprogramm aufruft, die tragbare Vorrichtung (100, 1700) veranlasst wird, das Verfahren nach einem der Ansprüche 1 bis 13 auszuführen.

15. Computerlesbares Speichermedium, das dazu konfiguriert ist, ein Computerprogramm zu speichern, wobei das Computerprogramm eine Anweisung zum Implementieren des Verfahrens nach einem der Ansprüche 1 bis 13 umfasst.

## Revendications

1. Procédé de mesure de la pression artérielle d'un dispositif portable (100, 1700), dans lequel le dispositif portable (100, 1700) comprend un transducteur de pression (120), PT, un écran d'affichage (161) et une photodiode, PD, (131) ; et
le procédé comprend :
l'émission d'un signal optique à travers une première région de l'écran d'affichage (161) ;
la conversion, en un premier signal PPG, du signal optique reçu par la PD (131), dans laquelle le signal optique émis par la première région est absorbé et diffusé par le tissu cutané pour former un signal réfléchi, et la PD convertit le signal réfléchi en le premier signal PPG ;
la détermination du fait que le premier signal PPG satisfait ou non à une exigence de qualité de signal en calculant un taux de perfusion du premier signal PPG et en comparant le taux de perfusion à un taux de perfusion prédéfini ou en calculant une intensité du premier signal PPG et en comparant l'intensité à une intensité prédéfinie ;
si le premier signal PPG satisfait à l'exigence de qualité de signal :
l'obtention d'une valeur de pression collectée par le PT (120) ; et
la détermination d'une valeur de pression artérielle d'un porteur sur la base de la valeur de pression et du premier signal PPG ;
si le premier signal PPG ne satisfait pas à une exigence de qualité de signal :
l'émission du signal optique à travers une seconde région de l'écran d'affichage (161), dans laquelle la seconde région et la première région sont différentes dans au moins l'une des caractéristiques suivantes : une position, une luminance, une taille de zone ou une forme sur l'écran d'affichage (161) ;
la conversion, en un second signal PPG, du signal optique reçu par la PD (131) ;
la détermination du fait que le second signal PPG satisfait ou non à l'exigence de qualité de signal en calculant un taux de perfusion du second signal PPG et en comparant le taux de perfusion au taux de perfusion prédéfini ou en calculant une intensité du second signal PPG et en comparant l'intensité à une intensité prédéfinie ;
si le second signal PPG satisfait à l'exigence de qualité de signal :
l'obtention d'une valeur de pression collectée par le PT (120) lorsque le second signal PPG satisfait à l'exigence de qualité de signal ; et
la détermination d'une valeur de pression artérielle d'un porteur sur la base de la valeur de pression et du second signal PPG.

2. Procédé selon la revendication 1, dans lequel, si le premier signal PPG ne satisfait pas à une exigence de qualité de signal comprend : un taux de perfusion du premier signal PPG est inférieur ou égal à un taux de perfusion prédéfini,
une distance entre une région de projection du PD (131) sur l'écran d'affichage (161) et la deuxième région est supérieure à une distance entre la région de projection du PD (131) sur l'écran d'affichage (161) et la première région.

3. Procédé selon la revendication 1, dans lequel, si le premier signal PPG ne satisfait pas à une exigence de qualité de signal comprend : une intensité du premier signal PPG est inférieure ou égale à une intensité prédéfinie,
la luminance de la deuxième région est supérieure à la luminance de la première région.

4. Procédé selon la revendication 3, dans lequel la luminance de la deuxième région est une valeur maximale ; et
le procédé comprend en outre :
l'émission du signal optique à travers une troisième région de l'écran d'affichage (161) si le deuxième signal PPG ne satisfait pas à l'exigence de qualité de signal, dans lequel une distance entre une région de projection du PD (131) sur l'écran d'affichage (161) et la troisième région est inférieure à une distance entre la région de projection du PD (131) sur l'écran d'affichage (161) et la première région ;
la conversion, en un troisième signal PPG, du signal optique reçu par le PD (131) ;
l'obtention de la valeur de pression collectée par le PT (120) lorsque le troisième signal PPG satisfait à l'exigence de qualité de signal ; et
la détermination de la valeur de pression artérielle du porteur sur la base de la valeur de pression et du troisième signal PPG.

5. Procédé selon la revendication 1, dans lequel la forme de la deuxième région est différente de la forme de la première région ; ou
la taille de la surface de la deuxième région est différente de la taille de la surface de la première région.

6. Procédé selon l'une quelconque des revendications 1 à 5, dans lequel la valeur de pression comprend une pluralité de valeurs de pression, le deuxième signal PPG comprend une pluralité de deuxièmes signaux PPG, la pluralité de valeurs de pression sont en correspondance biunivoque avec la pluralité de deuxièmes signaux PPG, et un moment de collecte de chacune de la pluralité de valeurs de pression est identique à celui d'un signal correspondant des deuxièmes signaux PPG ; et
la détermination d'une valeur de pression artérielle d'un porteur sur la base de la valeur de pression et du deuxième signal PPG comprend :
l'extraction d'une caractéristique de forme d'onde de chacun de la pluralité de deuxièmes signaux PPG ; et
la détermination, si un deuxième signal PPG cible dont la caractéristique de forme d'onde est inférieure ou égale à une caractéristique de forme d'onde prédéfinie existe dans la pluralité de deuxièmes signaux PPG, d'une valeur de pression correspondant au deuxième signal PPG cible en tant que pression artérielle systolique, PAS, dans la valeur de pression artérielle du porteur.

7. Procédé selon l'une quelconque des revendications 1 à 6, dans lequel la valeur de pression comprend une pluralité de valeurs de pression, le second signal PPG comprend une pluralité de seconds signaux PPG, la pluralité de valeurs de pression sont en correspondance biunivoque avec la pluralité de seconds signaux PPG, et un moment de collecte de chacune de la pluralité de valeurs de pression est identique à celui d'un signal correspondant parmi les seconds signaux PPG ; et
la détermination d'une valeur de pression artérielle d'un porteur sur la base de la valeur de pression et du second signal PPG comprend :
l'extraction d'une caractéristique de forme d'onde de chacun de la pluralité de seconds signaux PPG ; et
l'introduction de la pluralité de valeurs de pression et des caractéristiques de forme d'onde de la pluralité de seconds signaux PPG dans un modèle de réseau neuronal, dans lequel le modèle de réseau neuronal est entraîné sur la base d'une valeur de pression historique et d'une caractéristique de forme d'onde d'un signal PPG historique, et est configuré pour mesurer la pression artérielle ; et
la détermination d'une sortie du modèle de réseau neuronal en tant que pression artérielle diastolique DBP dans la valeur de pression artérielle du porteur.

8. Procédé selon l'une quelconque des revendications 1 à 7, dans lequel avant l'obtention d'une valeur de pression collectée par le PT (120), le procédé comprend en outre :
la sortie d'un guide de manipulation, dans lequel le guide de manipulation est configuré pour guider le porteur pour appliquer une pression perpendiculaire à une surface de contact entre le dispositif portable (100, 1700) et la peau humaine au dispositif portable (100, 1700) dans une zone de pression prédéfinie, et le guide de manipulation comprend une valeur de pression requise pour que le porteur appuie.

9. Procédé selon la revendication 8, comprenant en outre :
l'arrêt, pendant l'application de pression par le porteur, de la mesure de pression artérielle si la condition suivante est satisfaite :
une accélération du dispositif portable (100, 1700) est supérieure à un seuil d'accélération ; et/ou
une vitesse angulaire du dispositif portable (100, 1700) est supérieure à un seuil de vitesse angulaire.

10. Procédé selon la revendication 8 ou 9, comprenant en outre :
l'affichage d'une première interface, dans lequel la première interface est configurée pour afficher une courbe de pression, des informations d'invite, et une forme d'onde du second signal PPG, la courbe de pression est le guide de manipulation, et les informations d'invite sont configurées pour inviter une durée de pression du porteur.

11. Procédé selon l'une quelconque des revendications 1 à 10, dans lequel avant l'émission d'un signal optique à travers une première région de l'écran d'affichage (161), le procédé comprend en outre :
le fait de déterminer si le dispositif portable (100, 1700) satisfait à une condition de mesure de pression artérielle ; et
l'émission d'un signal optique à travers une première région de l'écran d'affichage (161) comprend :
émettre le signal optique à travers la première région de l'écran d'affichage (161) lorsque la condition de mesure de la pression artérielle est satisfaite.

12. Procédé selon la revendication 11, dans lequel la condition de mesure de la pression artérielle comprend au moins l'un des éléments suivants :
l'accélération du dispositif portable (100, 1700) est inférieure ou égale au seuil d'accélération, la valeur de pression est supérieure ou égale à un seuil de pression, ou la vitesse angulaire du dispositif portable (100, 1700) est inférieure ou égale au seuil de vitesse angulaire, dans lequel
l'accélération est configurée pour représenter une situation de contact entre le dispositif portable (100, 1700) et le porteur, et la valeur de pression est configurée pour représenter un serrage pour le port du dispositif portable (100, 1700).

13. Procédé selon l'une quelconque des revendications 1 à 12, dans lequel l'écran d'affichage (161) est supporté par un support d'écran, le support d'écran comprend une fenêtre optique, le PD (131) se trouve directement sous la fenêtre optique, une distance prédéfinie est définie entre le PD (131) et la fenêtre optique, le PT (120) est situé au niveau d'une partie inférieure du dispositif portable (100, 1700) et est configuré pour collecter une valeur de pression entre le dispositif portable (100, 1700) et une partie de port, et la partie de port est une partie de contact entre le dispositif portable (100, 1700) et le porteur.

14. Dispositif portable (100, 1700), comprenant : un processeur (1710), dans lequel le processeur (1710) est couplé à une mémoire (1730), la mémoire (1730) est configurée pour stocker un programme informatique, et lorsque le processeur (1710) appelle le programme informatique, le dispositif portable (100, 1700) est amené à mettre en œuvre le procédé selon l'une quelconque des revendications 1 à 13.

15. Support de stockage lisible par ordinateur, configuré pour stocker un programme informatique, dans lequel le programme informatique comprend une instruction pour mettre en œuvre le procédé selon l'une quelconque des revendications 1 à 13.
